# EUROPEAN PATENT APPLICATION

(11) **EP 0 874 045 A1**
(43) Date of publication of application: **28.10.1998**
(21) Application number: 97935810.8
(22) Date of filing: 19.08.1997
(51) Int. Cl.: C12N 15/00, C12P 21/00

(54) **NOVEL DNAS AND PROCESS FOR PRODUCING PROTEINS BY USING THE SAME**

(30) Priority: 19.08.1996 JP 235928/96
(71) Applicant: SNOW BRAND MILK PRODUCTS CO., LTD., Sapporo-shi, Hokkaido 065 (JP)
(72) Inventor: NAKAGAWA, Nobuaki, Nishiura Heights 2-4, Shimotsuga-gun, Tochigi 329-05 (JP); YASUDA, Hisataka, Kawachi-gun, Tochigi 329-04 (JP); MORINAGA, Tomonori, Shimotsuga-gun, Tochigi 321-02 (JP)
(74) Representative: Wakerley, Helen Rachael
(86) International application number: JP9702859
(87) International publication number: WO9807840

(57) **Abstract**

DNAs having the nucleotide sequences of the Sequences No. 1 and No. 2 in the Sequence Table and a process for producing a protein which comprises inserting these DNAs into expression vectors to thereby produce a protein having molecular weights of about 60 kD (under reductive conditions) and about 60 kD and 120 kD (under non-reductive conditions) and being capable of inhibiting formation of osteoclast. These proteins are useful in the treatment of osteoporosis and rheumatism.

SEQUENCE LISTING

(1) GENERAL INFORMATION:

(i) APPLICANT:
(A) NAME: SNOW BRAND MILK PRODUCTS CO., LTD.
(B) STREET: 1-1, NAEBOCHO 6-CHOME
(C) CITY: HIGASHI-KU, SAPPORO-SHI
(D) STATE: HOKKAIDO
(E) COUNTRY: JP
(F) POSTAL CODE (ZIP): NONE

(ii) TITLE OF INVENTION: NOVEL DNA AND PROCESS FOR PREPARING PROTEIN
USING THE DNA

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(v) CURRENT APPLICATION DATA:
APPLICATION NUMBER: EP 97935810.8
(vi) PRIOR APPLICATION DATA:
(A) APPLICATION NUMBER: JP 235928/96
(B) FILING DATE: 19-AUG-1996

(2) INFORMATION FOR SEQ ID NO:1:

(i) SEQUENCE CHARACTERISTICS:
(A) LENGTH: 1316 base pairs
(B) TYPE: nucleic acid
(C) STRANDEDNESS: double
(D) TOPOLOGY: linear
(ii) MOLECULE TYPE: genomic DNA (human OCIF genomic DNA-1)

(xi) SEQUENCE DESCRIPTION: SEQ ID NO:1:

CTGGAGACAT ATAACTTGAA CACTTGGCCC TGATGGGGAA GCAGCTCTGC AGGGACTTTT 60
TCAGCCATCT GTAAACAATT TCAGTGGCAA CCCGCGAACT GTAATCCATG AATGGGACCA 120
CACTTTACAA GTCATCAAGT CTAACTTCTA GACCAGGGAA TTAATGGGGG AGACAGCGAA 180
CCCTAGAGCA AAGTGCCAAA CTTCTGTCGA TAGCTTGAGG CTAGTGGAAA GACCTCGAGG 240
AGGCTACTCC AGAAGTTCAG CGCGTAGGAA GCTCCGATAC CAATAGCCCT TTGATGATGG 300
TGGGGTTGGT GAAGGGAACA GTGCTCCGCA AGGTTATCCC TGCCCCAGGC AGTCCAATTT 360
TCACTCTGCA GATTCTCTCT GGCTCTAACT ACCCCAGATA ACAAGGAGTG AATGCAGAAT 420
AGCACGGGCT TTAGGGCCAA TCAGACATTA GTTAGAAAAA TTCCTACTAC ATGGTTTATG 480
TAAACTTGAA GATGAATGAT TGCGAACTCC CCGAAAAGGG CTCAGACAAT GCCATGCATA 540
AAGAGGGGCC CTGTAATTTG AGGTTTCAGA ACCCGAAGTG AAGGGGTCAG GCAGCCGGGT 600
ACGGCGGAAA CTCACAGCTT TCGCCCAGCG AGAGGACAAA GGTCTGGGAC ACACTCCAAC 660
TGCGTCCGGA TCTTGGCTGG ATCGGACTCT CAGGGTGGAG GAGACACAAG CACAGCAGCT 720
GCCCAGCGTG TGCCCAGCCC TCCCACCGCT GGTCCCGGCT GCCAGGAGGC TGGCCGCTGG 780
CGGGAAGGGG CCGGGAAACC TCAGAGCCCC GCGGAGACAG CAGCCGCCTT GTTCCTCAGC 840
CCGGTGGCTT TTTTTTCCCC TGCTCTCCCA GGGGACAGAC ACCACCGCCC CACCCCTCAC 900
GCCCCACCTC CCTGGGGGAT CCTTTCCGCC CCAGCCCTGA AAGCGTTAAT CCTGGAGCTT 960
TCTGCACACC CCCCGACCGC TCCCGCCCAA GCTTCCTAAA AAAGAAAGGT GCAAAGTTTG 1020
GTCCAGGATA GAAAAATGAC TGATCAAAGG CAGGCGATAC TTCCTGTTGC CGGGACGCTA 1080
TATATAACGT GATGAGCGCA CGGGCTGCGG AGACGCACCG GAGCGCTCGC CCAGCCGCCG 1140
CCTCCAAGCC CCTGAGGTTT CCGGGGACCA CA ATG AAC AAG TTG CTG TGC TGC 1193
Met Asn Lys Leu Leu Cys Cys
-20 -15

GCG CTC GTG GTAAGTCCCT GGGCCAGCCG ACGGGTGCCC GGCGCCTGGG 1242

Ala Leu Val

GAGGCTGCTG CCACCTGGTC TCCCAACCTC CCAGCGGACC GGCGGGGAGA AGGCTCCACT 1302
CGCTCCCTCC CAGG 1316

(2) INFORMATION FOR SEQ ID NO:2:

(i) SEQUENCE CHARACTERISTICS:
(A) LENGTH: 9898 base pairs
(B) TYPE: nucleic acid
(C) STRANDEDNESS: double
(D) TOPOLOGY: linear
(ii) MOLECULE TYPE: genomic DNA (human OCIF genomic DNA-2)

(xi) SEQUENCE DESCRIPTION: SEQ ID NO:2:

GCTTACTTTG TGCCAAATCT CATTAGGCTT AAGGTAATAC AGGACTTTGA GTCAAATGAT 60
ACTGTTGCAC ATAAGAACAA ACCTATTTTC ATGCTAAGAT GATGCCACTG TGTTCCTTTC 120
TCCTTCTAG TTT CTG GAC ATC TCC ATT AAG TGG ACC ACC CAG GAA ACG TTT 171
Phe Leu Asp Ile Ser Ile Lys Trp Thr Thr Gln Glu Thr Phe
-10 -5 1

CCT CCA AAG TAC CTT CAT TAT GAC GAA GAA ACC TCT CAT CAG CTG TTG 219
Pro Pro Lys Tyr Leu His Tyr Asp Glu Glu Thr Ser His Gln Leu Leu
                5                   10                  15

TGT GAC AAA TGT CCT CCT GGT ACC TAC CTA AAA CAA CAC TGT ACA GCA 267
Cys Asp Lys Cys Pro Pro Gly Thr Tyr Leu Lys Gln His Cys Thr Ala
20 25 30 35

AAG TGG AAG ACC GTG TGC GCC CCT TGC CCT GAC CAC TAC TAC ACA GAC 315
Lys Trp Lys Thr Val Cys Ala Pro Cys Pro Asp His Tyr Tyr Thr Asp
40 45 50

AGC TGG CAC ACC AGT GAC GAG TGT CTA TAC TGC AGC CCC GTG TGC AAG 363
Ser Trp His Thr Ser Asp Glu Cys Leu Tyr Cys Ser Pro Val Cys Lys
55 60 65

GAG CTG CAG TAC GTC AAG CAG GAG TGC AAT CGC ACC CAC AAC CGC GTG 411
Glu Leu Gln Tyr Val Lys Gln Glu Cys Asn Arg Thr His Asn Arg Val
70 75 80

TGC GAA TGC AAG GAA GGG CGC TAC CTT GAG ATA GAG TTC TGC TTG AAA 459
Cys Glu Cys Lys Glu Gly Arg Tyr Leu Glu Ile Glu Phe Cys Leu Lys
                85                  90                  95

CAT AGG AGC TGC CCT CCT GGA TTT GGA GTG GTG CAA GCT G GTACGTGTCA 509
His Arg Ser Cys Pro Pro Gly Phe Gly Val Val Gln Ala
100 105 110

ATGTGCAGCA AAATTAATTA GGATCATGCA AAGTCAGATA GTTGTGACAG TTTAGGAGAA 569
CACTTTTGTT CTGATGACAT TATAGGATAG CAAATTGCAA AGGTAATGAA ACCTGCCAGG 629
TAGGTACTAT GTGTCTGGAG TGCTTCCAAA GGACCATTGC TCAGAGGAAT ACTTTGCCAC 689
TACAGGGCAA TTTAATGACA AATCTCAAAT GCAGCAAATT ATTCTCTCAT GAGATGCATG 749
ATGGTTTTTT TTTTTTTTTT TAAAGAAACA AACTCAAGTT GCACTATTGA TAGTTGATCT 809
ATACCTCTAT ATTTCACTTC AGCATGGACA CCTTCAAACT GCAGCACTTT TTGACAAACA 869
TCAGAAATGT TAATTTATAC CAAGAGAGTA ATTATGCTCA TATTAATGAG ACTCTGGAGT 929
GCTAACAATA AGCAGTTATA ATTAATTATG TAAAAAATGA GAATGGTGAG GGGAATTGCA 989
TTTCATTATT AAAAACAAGG CTAGTTCTTC CTTTAGCATG GGAGCTGAGT GTTTGGGAGG 1049
GTAAGGACTA TAGCAGAATC TCTTCAATGA GCTTATTCTT TATCTTAGAC AAAACAGATT 1109
GTCAAGCCAA GAGCAAGCAC TTGCCTATAA ACCAAGTGCT TTCTCTTTTG CATTTTGAAC 1169
AGCATTGGTC AGGGCTCATG TGTATTGAAT CTTTTAAACC AGTAACCCAC GTTTTTTTTC 1229
TGCCACATTT GCGAAGCTTC AGTGCAGCCT ATAACTTTTC ATAGCTTGAG AAAATTAAGA 1289
GTATCCACTT ACTTAGATGG AAGAAGTAAT CAGTATAGAT TCTGATGACT CAGTTTGAAG 1349

CAGTGTTTCT CAACTGAAGC CCTGCTGATA TTTTAAGAAA TATCTGGATT CCTAGGCTGG 1409
ACTCCTTTTT GTGGGCAGCT GTCCTGCGCA TTGTAGAATT TTGGCAGCAC CCCTGGACTC 1469
TAGCCACTAG ATACCAATAG CAGTCCTTCC CCCATGTGAC AGCCAAAAAT GTCTTCAGAC 1529
ACTGTCAAAT GTCGCCAGGT GGCAAAATCA CTCCTGGTTG AGAACAGGGT CATCAATGCT 1589
AAGTATCTGT AACTATTTTA ACTCTCAAAA CTTGTGATAT ACAAAGTCTA AATTATTAGA 1649
CGACCAATAC TTTAGGTTTA AAGGCATACA AATGAAACAT TCAAAAATCA AAATCTATTC 1709
TGTTTCTCAA ATAGTGAATC TTATAAAATT AATCACAGAA GATGCAAATT GCATCAGAGT 1769
CCCTTAAAAT TCCTCTTCGT ATGAGTATTT GAGGGAGGAA TTGGTGATAG TTCCTACTTT 1829
CTATTGGATG GTACTTTGAG ACTCAAAAGC TAAGCTAAGT TGTGTGTGTG TCAGGGTGCG 1889
GGGTGTGGAA TCCCATCAGA TAAAAGCAAA TCCATGTAAT TCATTCAGTA AGTTGTATAT 1949
GTAGAAAAAT GAAAAGTGGG CTATGCAGCT TGGAAACTAG AGAATTTTGA AAAATAATGG 2009
AAATCACAAG GATCTTTCTT AAATAAGTAA GAAAATCTGT TTGTAGAATG AAGCAAGCAG 2069
GCAGCCAGAA GACTCAGAAC AAAAGTACAC ATTTTACTCT GTGTACACTG GCAGCACAGT 2129
GGGATTTATT TACCTCTCCC TCCCTAAAAA CCCACACAGC GGTTCCTCTT GGGAAATAAG 2189
AGGTTTCCAG CCCAAAGAGA AGGAAAGACT ATGTGGTGTT ACTCTAAAAA GTATTTAATA 2249
ACCGTTTTGT TGTTGCTGTT GCTGTTTTGA AATCAGATTG TCTCCTCTCC ATATTTTATT 2309
TACTTCATTC TGTTAATTCC TGTGGAATTA CTTAGAGCAA GCATGGTGAA TTCTCAACTG 2369
TAAAGCCAAA TTTCTCCATC ATTATAATTT CACATTTTGC CTGGCAGGTT ATAATTTTTA 2429
TATTTCCACT GATAGTAATA AGGTAAAATC ATTACTTAGA TGGATAGATC TTTTTCATAA 2489
AAAGTACCAT CAGTTATAGA GGGAAGTCAT GTTCATGTTC AGGAAGGTCA TTAGATAAAG 2549
CTTCTGAATA TATTATGAAA CATTAGTTCT GTCATTCTTA GATTCTTTTT GTTAAATAAC 2609
TTTAAAAGCT AACTTACCTA AAAGAAATAT CTGACACATA TGAACTTCTC ATTAGGATGC 2669
AGGAGAAGAC CCAAGCCACA GATATGTATC TGAAGAATGA ACAAGATTCT TAGGCCCGGC 2729
ACGGTGGCTC ACATCTGTAA TCTCAAGAGT TTGAGAGGTC AAGGCGGGCA GATCACCTGA 2789
GGTCAGGAGT TCAAGACCAG CCTGGCCAAC ATGATGAAAC CCTGCCTCTA CTAAAAATAC 2849
AAAAATTAGC AGGGCATGGT GGTGCATGCC TGCAACCCTA GCTACTCAGG AGGCTGAGAC 2909
AGGAGAATCT CTTGAACCCT CGAGGCGGAG GTTGTGGTGA GCTGAGATCC CTCTACTGCA 2969
CTCCAGCCTG GGTGACAGAG ATGAGACTCC GTCCCTGCCG CCGCCCCCGC CTTCCCCCCC 3029
AAAAAGATTC TTCTTCATGC AGAACATACG GCAGTCAACA AAGGGAGACC TGGGTCCAGG 3089
TGTCCAAGTC ACTTATTTCG AGTAAATTAG CAATGAAAGA ATGCCATGGA ATCCCTGCCC 3149
AAATACCTCT GCTTATGATA TTGTAGAATT TGATATAGAG TTGTATCCCA TTTAAGGAGT 3209
AGGATGTAGT AGGAAAGTAC TAAAAACAAA CACACAAACA GAAAACCCTC TTTGCTTTGT 3269
AAGGTGGTTC CTAAGATAAT GTCAGTGCAA TGCTGGAAAT AATATTTAAT ATGTGAAGGT 3329
TTTAGGCTGT GTTTTCCCCT CCTGTTCTTT TTTTCTGCCA GCCCTTTGTC ATTTTTGCAG 3389
GTCAATGAAT CATGTAGAAA GAGACAGGAG ATGAAACTAG AACCAGTCCA TTTTGCCCCT 3449
TTTTTTATTT TCTGGTTTTG GTAAAAGATA CAATGAGGTA GGAGGTTGAG ATTTATAAAT 3509
GAAGTTTAAT AAGTTTCTGT AGCTTTGATT TTTCTCTTTC ATATTTGTTA TCTTGCATAA 3569
GCCAGAATTG GCCTGTAAAA TCTACATATG GATATTGAAG TCTAAATCTG TTCAACTAGC 3629
TTACACTAGA TGGAGATATT TTCATATTCA GATACACTGG AATGTATGAT CTAGCCATGC 3689
GTAATATAGT CAAGTGTTTG AAGGTATTTA TTTTTAATAG CGTCTTTAGT TGTGGACTGG 3749
TTCAAGTTTT TCTGCCAATG ATTTCTTCAA ATTTATCAAA TATTTTTCCA TCATGAAGTA 3809
AAATGCCCTT GCAGTCACCC TTCCTGAAGT TTGAACGACT CTGCTGTTTT AAACAGTTTA 3869
AGCAAATGGT ATATCATCTT CCGTTTACTA TGTAGCTTAA CTGCAGGCTT ACGCTTTTGA 3929
GTCAGCGGCC AACTTTATTG CCACCTTCAA AAGTTTATTA TAATGTTGTA AATTTTTACT 3989
TCTCAAGGTT AGCATACTTA GGAGTTGCTT CACAATTAGG ATTCAGGAAA GAAAGAACTT 4049
CAGTAGGAAC TGATTGGAAT TTAATGATGC AGCATTCAAT GGGTACTAAT TTCAAAGAAT 4109
GATATTACAG CAGACACACA GCAGTTATCT TGATTTTCTA GGAATAATTG TATGAAGAAT 4169
ATGGCTGACA ACACGGCCTT ACTGCCACTC AGCGGAGGCT GGACTAATGA ACACCCTACC 4229
CTTCTTTCCT TTCCTCTCAC ATTTCATGAG CGTTTTGTAG GTAACGAGAA AATTGACTTG 4289
CATTTGCATT ACAAGGAGGA GAAACTGGCA AAGGGGATGA TGGTGGAAGT TTTGTTCTGT 4349
CTAATGAAGT GAAAAATGAA AATGCTAGAG TTTTGTGCAA CATAATAGTA GCAGTAAAAA 4409
CCAAGTGAAA AGTCTTTCCA AAACTGTGTT AAGAGGGCAT CTGCTGGGAA ACGATTTGAG 4469
GAGAAGGTAC TAAATTGCTT GGTATTTTCC GTAG GA ACC CCA GAG CGA AAT ACA 4523
Gly Thr Pro Glu Arg Asn Thr
115

GTT TGC AAA AGA TGT CCA GAT GGG TTC TTC TCA AAT GAG ACG TCA TCT 4571
Val Cys Lys Arg Cys Pro Asp Gly Phe Phe Ser Asn Glu Thr Ser Ser
120 125 130 135

AAA GCA CCC TGT AGA AAA CAC ACA AAT TGC AGT GTC TTT GGT CTC CTG 4619
Lys Ala Pro Cys Arg Lys His Thr Asn Cys Ser Val Phe Gly Leu Leu
140 145 150

CTA ACT CAG AAA GGA AAT GCA ACA CAC GAC AAC ATA TGT TCC GGA AAC 4667

Leu Thr Gln Lys Gly Asn Ala Thr His Asp Asn Ile Cys Ser Gly Asn
        155                 160                 165

AGT GAA TCA ACT CAA AAA TGT GGA ATA G GTAATTACAT TCCAAAATAC      4715
Ser Glu Ser Thr Gln Lys Cys Gly Ile
    170                 175

GTCTTTGTAC GATTTTGTAG TATCATCTCT CTCTCTGAGT TGAACACAAG GCCTCCAGCC 4775
ACATTCTTGG TCAAACTTAC ATTTTCCCTT TCTTGAATCT TAACCAGCTA AGGCTACTCT 4835
CGATGCATTA CTGCTAAAGC TACCACTCAG AATCTCTCAA AAACTCATCT TCTCACAGAT 4895
AACACCTCAA AGCTTGATTT TCTCTCCTTT CACACTGAAA TCAAATCTTG CCCATAGGCA 4955
AAGGGCAGTG TCAAGTTTGC CACTGAGATG AAATTAGGAG AGTCCAAACT GTAGAATTCA 5015
CGTTGTGTGT TATTACTTTC ACGAATGTCT GTATTATTAA CTAAAGTATA TATTGGCAAC 5075
TAAGAAGCAA AGTGATATAA ACATGATGAC AAATTAGGCC AGGCATGGTG GCTTACTCCT 5135
ATAATCCCAA CATTTTGGGG GGCCAAGGTA GGCAGATCAC TTGAGGTCAG GATTTCAAGA 5195
CCAGCCTGAC CAACATGGTG AAACCTTGTC TCTACTAAAA ATACAAAAAT TAGCTGGGCA 5255
TGGTAGCAGG CACTTCTAGT ACCAGCTACT CAGGGCTGAG GCAGGAGAAT CGCTTGAACC 5315
CAGGAGATGG AGGTTGCAGT GAGCTGAGAT TGTACCACTG CACTCCAGTC TGGGCAACAG 5375
AGCAAGATTT CATCACACAC ACACACACAC ACACACACAC ACACATTAGA AATGTGTACT 5435
TGGCTTTGTT ACCTATGGTA TTAGTGCATC TATTGCATGG AACTTCCAAG CTACTCTGGT 5495
TGTGTTAAGC TCTTCATTGG GTACAGGTCA CTAGTATTAA GTTCAGGTTA TTCGGATGCA 5555
TTCCACGGTA GTGATGACAA TTCATCAGGC TAGTGTGTGT GTTCACCTTG TCACTCCCAC 5615
CACTAGACTA ATCTCAGACC TTCACTCAAA GACACATTAC ACTAAAGATG ATTTGCTTTT 5675
TTGTGTTTAA TCAAGCAATG GTATAAACCA GCTTGACTCT CCCCAAACAG TTTTTCGTAC 5735
TACAAAGAAG TTTATGAAGC AGAGAAATGT GAATTGATAT ATATATGAGA TTCTAACCCA 5795
GTTCCAGCAT TGTTTCATTG TGTAATTGAA ATCATAGACA AGCCATTTTA GCCTTTGCTT 5855
TCTTATCTAA AAAAAAAAAA AAAAAAATGA AGGAAGGGGT ATTAAAAGGA GTGATCAAAT 5915
TTTAACATTC TCTTTAATTA ATTCATTTTT AATTTTACTT TTTTTCATTT ATTGTGCACT 5975
TACTATGTGG TACTGTGCTA TAGAGGCTTT AACATTTATA AAAACACTGT GAAAGTTGCT 6035
TCAGATGAAT ATAGGTAGTA GAACGGCAGA ACTAGTATTC AAAGCCAGGT CTGATGAATC 6095
CAAAAACAAA CACCCATTAC TCCCATTTTC TGGGACATAC TTACTCTACC CAGATGCTCT 6155
GGGCTTTGTA ATGCCTATGT AAATAACATA GTTTTATGTT TGGTTATTTT CCTATGTAAT 6215
GTCTACTTAT ATATCTGTAT CTATCTCTTG CTTTGTTTCC AAAGGTAAAC TATGTGTCTA 6275
AATGTGGGCA AAAAATAACA CACTATTCCA AATTACTGTT CAAATTCCTT TAAGTCAGTG 6335
ATAATTATTT GTTTTGACAT TAATCATGAA GTTCCCTGTG GGTACTAGGT AAACCTTTAA 6395
TAGAATGTTA ATGTTTGTAT TCATTATAAG AATTTTTGGC TGTTACTTAT TTACAACAAT 6455
ATTTCACTCT AATTAGACAT TTACTAAACT TTCTCTTGAA AACAATGCCC AAAAAAGAAC 6515
ATTAGAAGAC ACGTAAGCTC AGTTGGTCTC TGCCACTAAG ACCAGCCAAC AGAAGCTTGA 6575
TTTTATTCAA ACTTTGCATT TTAGCATATT TTATCTTGGA AAATTCAATT GTGTTGGTTT 6635
TTTGTTTTTG TTTGTATTGA ATAGACTCTC AGAAATCCAA TTGTTGAGTA AATCTTCTGG 6695
GTTTTCTAAC CTTTCTTTAG AT GTT ACC CTG TGT GAG GAG GCA TTC TTC AGG 6747
                      Asp Val Thr Leu Cys Glu Glu Ala Phe Phe Arg
                                  180                 185

TTT GCT GTT CCT ACA AAG TTT ACG CCT AAC TGG CTT AGT GTC TTG GTA    6795
Phe Ala Val Pro Thr Lys Phe Thr Pro Asn Trp Leu Ser Val Leu Val
        190                 195                 200

GAC AAT TTG CCT GGC ACC AAA GTA AAC GCA GAG AGT GTA GAG AGG ATA    6843
Asp Asn Leu Pro Gly Thr Lys Val Asn Ala Glu Ser Val Glu Arg Ile
    205                 210                 215

AAA CGG CAA CAC AGC TCA CAA GAA CAG ACT TTC CAG CTG CTG AAG TTA    6891
Lys Arg Gln His Ser Ser Gln Glu Gln Thr Phe Gln Leu Leu Lys Leu
220                 225                 230                 235

TGG AAA CAT CAA AAC AAA GAC CAA GAT ATA GTC AAG AAG ATC ATC CAA G 6940
Trp Lys His Gln Asn Lys Asp Gln Asp Ile Val Lys Lys Ile Ile Gln
                240                 245                 250

GTATGATAAT CTAAAATAAA AAGATCAATC AGAAATCAAA GACACCTATT TATCATAAAC 7000
CAGGAACAAG ACTGCATGTA TGTTTAGTTG TGTGGATCTT GTTTCCCTGT TGGAATCATT 7060
GTTGGACTGA AAAAGTTTCC ACCTGATAAT GTAGATGTGA TTCCACAAAC AGTTATACAA 7120
GGTTTTGTTC TCACCCCTGC TCCCCAGTTT CCTTGTAAAG TATGTTGAAC ACTCTAAGAG 7180
AAGAGAAATG CATTTGAAGG CAGGGCTGTA TCTCAGGGAG TCGCTTCCAG ATCCCTTAAC 7240

GCTTCTGTAA GCAGCCCCTC TAGACCACCA AGGAGAAGCT CTATAACCAC TTTGTATCTT 7300
ACATTGCACC TCTACCAAGA AGCTCTGTTG TATTTACTTG GTAATTCTCT CCAGGTAGGC 7360
TTTTCGTAGC TTACAAATAT GTTCTTATTA ATCCTCATGA TATGGCCTGC ATTAAAATTA 7420
TTTTAATGGC ATATGTTATG AGAATTAATG AGATAAAATC TGAAAAGTGT TTGAGCCTCT 7480
TGTAGGAAAA AGCTAGTTAC AGCAAAATGT TCTCACATCT TATAAGTTTA TATAAAGATT 7540
CTCCTTTAGA AATGGTGTGA GAGAGAAACA GAGAGAGATA GGGAGAGAAG TGTGAAAGAA 7600
TCTGAAGAAA AGGAGTTTCA TCCAGTGTGG ACTGTAAGCT TTACGACACA TGATGGAAAG 7660
AGTTCTGACT TCAGTAAGCA TTGGGAGGAC ATGCTAGAAG AAAAAGGAAG AAGAGTTTCC 7720
ATAATGCAGA CAGGGTCAGT GAGAAATTCA TTCAGGTCCT CACCAGTAGT TAAATGACTG 7780
TATAGTCTTG CACTACCCTA AAAAACTTCA AGTATCTGAA ACCGGGGCAA CAGATTTTAG 7840
GAGACCAACG TCTTTGAGAG CTGATTGCTT TTGCTTATGC AAAGAGTAAA CTTTTATGTT 7900
TTGAGCAAAC CAAAAGTATT CTTTGAACGT ATAATTAGCC CTGAAGCCGA AAGAAAAGAG 7960
AAAATCAGAG ACCGTTAGAA TTGGAAGCAA CCAAATTCCC TATTTTATAA ATGAGGACAT 8020
TTTAACCCAG AAAGATGAAC CGATTTGGCT TAGGGCTCAC AGATACTAAG TGACTCATGT 8080
CATTAATAGA AATGTTAGTT CCTCCCTCTT AGGTTTGTAC CCTAGCTTAT TACTGAAATA 8140
TTCTCTAGGC TGTGTGTCTC CTTTAGTTCC TCGACCTCAT GTCTTTGAGT TTTCAGATAT 8200
CCTCCTCATG GAGGTAGTCC TCTGGTGCTA TGTGTATTCT TTAAAGGCTA GTTACGGCAA 8260
TTAACTTATC AACTAGCGCC TACTAATGAA ACTTTGTATT ACAAAGTAGC TAACTTGAAT 8320
ACTTTCCTTT TTTTCTGAAA TGTTATGGTG GTAATTTCTC AAACTTTTTC TTAGAAAACT 8380
GAGAGTGATG TGTCTTATTT TCTACTGTTA ATTTTCAAAA TTAGGAGCTT CTTCCAAAGT 8440
TTTGTTGGAT GCCAAAAATA TATAGCATAT TATCTTATTA TAACAAAAAA TATTTATCTC 8500
AGTTCTTAGA AATAAATGGT GTCACTTAAC TCCCTCTCAA AAGAAAAGGT TATCATTGAA 8560
ATATAATTAT GAAATTCTGC AAGAACCTTT TGCCTCACGC TTGTTTTATG ATGGCATTGG 8620
ATGAATATAA ATGATGTGAA CACTTATCTG GGCTTTTGCT TTATGCAG AT ATT GAC 8676
Asp Ile Asp

CTC TGT GAA AAC AGC GTG CAG CGG CAC ATT GGA CAT GCT AAC CTC ACC 8724
Leu Cys Glu Asn Ser Val Gln Arg His Ile Gly His Ala Asn Leu Thr
255 260 265 270

TTC GAG CAG CTT CGT AGC TTG ATG GAA AGC TTA CCG GGA AAG AAA GTG 8772
Phe Glu Gln Leu Arg Ser Leu Met Glu Ser Leu Pro Gly Lys Lys Val
275 280 285

GGA GCA GAA GAC ATT GAA AAA ACA ATA AAG GCA TGC AAA CCC AGT GAC 8820
Gly Ala Glu Asp Ile Glu Lys Thr Ile Lys Ala Cys Lys Pro Ser Asp
290 295 300

CAG ATC CTG AAG CTG CTC AGT TTG TGG CGA ATA AAA AAT GGC GAC CAA 8868
Gln Ile Leu Lys Leu Leu Ser Leu Trp Arg Ile Lys Asn Gly Asp Gln
305 310 315

GAC ACC TTG AAG GGC CTA ATG CAC GCA CTA AAG CAC TCA AAG ACG TAC 8916
Asp Thr Leu Lys Gly Leu Met His Ala Leu Lys His Ser Lys Thr Tyr
                320                 325                 330

CAC TTT CCC AAA ACT GTC ACT CAG AGT CTA AAG AAG ACC ATC AGG TTC 8964
His Phe Pro Lys Thr Val Thr Gln Ser Leu Lys Lys Thr Ile Arg Phe
335 340 345 350

CTT CAC AGC TTC ACA ATG TAC AAA TTG TAT CAG AAG TTA TTT TTA GAA 9012
Leu His Ser Phe Thr Met Tyr Lys Leu Tyr Gln Lys Leu Phe Leu Glu
355 360 365

ATG ATA GGT AAC CAG GTC CAA TCA GTA AAA ATA AGC TGC TTA 9054
Met Ile Gly Asn Gln Val Gln Ser Val Lys Ile Ser Cys Leu
370 375 380

TAACTGGAAA TGGCCATTGA GCTGTTTCCT CACAATTGGC GAGATCCCAT GGATGAGTAA 9114
ACTGTTTCTC AGGCACTTGA GGCTTTCAGT GATATCTTTC TCATTACCAG TGACTAATTT 9174
TGCCACAGGG TACTAAAAGA AACTATGATG TGGAGAAAGG ACTAACATCT CCTCCAATAA 9234
ACCCCAAATG GTTAATCCAA CTGTCAGATC TGGATCGTTA TCTACTGACT ATATTTTCCC 9294
TTATTACTGC TTGCAGTAAT TCAACTGGAA ATTAAAAAAA AAAAACTAGA CTCCACTGGG 9354
CCTTACTAAA TATGGGAATG TCTAACTTAA ATAGCTTTGG GATTCCAGCT ATGCTAGAGG 9414
CTTTTATTAG AAAGCCATAT TTTTTTCTGT AAAAGTTACT AATATATCTG TAACACTATT 9474

ACAGTATTGC TATTTATATT CATTCAGATA TAAGATTTGG ACATATTATC ATCCTATAAA 9534
GAAACGGTAT GACTTAATTT TAGAAAGAAA ATTATATTCT GTTTATTATG ACAAATGAAA 9594
GAGAAAATAT ATATTTTTAA TGGAAAGTTT GTAGCATTTT TCTAATAGGT ACTGCCATAT 9654
TTTTCTGTGT GGAGTATTTT TATAATTTTA TCTGTATAAG CTGTAATATC ATTTTATAGA 9714
AAATGCATTA TTTAGTCAAT TGTTTAATGT TGGAAAACAT ATGAAATATA AATTATCTGA 9774
ATATTAGATG CTCTGAGAAA TTGAATGTAC CTTATTTAAA AGATTTTATG GTTTTATAAC 9834
TATATAAATG ACATTATTAA AGTTTTCAAA TTATTTTTTA TTGCTTTCTC TGTTGCTTTT 9894
ATTT 9898

(2) INFORMATION FOR SEQ ID NO:3:

(i) SEQUENCE CHARACTERISTICS:
(A) LENGTH: 401 amino acids
(B) TYPE: amino acid
(C) STRANDEDNESS: single
(D) TOPOLOGY: linear
(ii) MOLECULE TYPE: protein

(xi) SEQUENCE DESCRIPTION: SEQ ID NO:3:

Met Asn Asn Leu Leu Cys Cys Ala Leu Val Phe Leu Asp Ile Ser
-20 -15 -10
Ile Lys Trp Thr Thr Gln Glu Thr Phe Pro Pro Lys Tyr Leu His
-5 1 5
Tyr Asp Glu Glu Thr Ser His Gln Leu Leu Cys Asp Lys Cys Pro
10 15 20
Pro Gly Thr Tyr Leu Lys Gln His Cys Thr Ala Lys Trp Lys Thr
25 30 35
Val Cys Ala Pro Cys Pro Asp His Tyr Tyr Thr Asp Ser Trp His
40 45 50
Thr Ser Asp Glu Cys Leu Tyr Cys Ser Pro Val Cys Lys Glu Leu
55 60 65
Gln Tyr Val Lys Gln Glu Cys Asn Arg Thr His Asn Arg Val Cys
70 75 80
Glu Cys Lys Glu Gly Arg Tyr Leu Glu Ile Glu Phe Cys Leu Lys
85 90 95
His Arg Ser Cys Pro Pro Gly Phe Gly Val Val Gln Ala Gly Thr
100 105 110
Pro Glu Arg Asn Thr Val Cys Lys Arg Cys Pro Asp Gly Phe Phe
115 120 125
Ser Asn Glu Thr Ser Ser Lys Ala Pro Cys Arg Lys His Thr Asn
130 135 140
Cys Ser Val Phe Gly Leu Leu Leu Thr Gln Lys Gly Asn Ala Thr
145 150 155
His Asp Asn Ile Cys Ser Gly Asn Ser Glu Ser Thr Gln Lys Cys
160 165 170
Gly Ile Asp Val Thr Leu Cys Glu Glu Ala Phe Phe Arg Phe Ala
175 180 185
Val Pro Thr Lys Phe Thr Pro Asn Trp Leu Ser Val Leu Val Asp
190 195 200
Asn Leu Pro Gly Thr Lys Val Asn Ala Glu Ser Val Glu Arg Ile
205 210 215
Lys Arg Gln His Ser Ser Gln Glu Gln Thr Phe Gln Leu Leu Lys
220 225 230
Leu Trp Lys His Gln Asn Lys Asp Gln Asp Ile Val Lys Lys Ile
235 240 245
Ile Gln Asp Ile Asp Leu Cys Glu Asn Ser Val Gln Arg His Ile
250 255 260
Gly His Ala Asn Leu Thr Phe Glu Gln Leu Arg Ser Leu Met Glu
265 270 275
Ser Leu Pro Gly Lys Lys Val Gly Ala Glu Asp Ile Glu Lys Thr
280 285 290
Ile Lys Ala Cys Lys Pro Ser Asp Gln Ile Leu Lys Leu Leu Ser
295 300 305

Leu Trp Arg Ile Lys Asn Gly Asp Gln Asp Thr Leu Lys Gly Leu
310                 315                 320
Met His Ala Leu Lys His Ser Lys Thr Tyr His Phe Pro Lys Thr
325                 330                 335
Val Thr Gln Ser Leu Lys Lys Thr Ile Arg Phe Leu His Ser Phe
340                 345                 350
Thr Met Tyr Lys Leu Tyr Gln Lys Leu Phe Leu Glu Met Ile Gly
355                 360                 365
Asn Gln Val Gln Ser Val Lys Ile Ser Cys Leu
370                 375                 380

(2) INFORMATION FOR SEQ ID NO:4:

(i) SEQUENCE CHARACTERISTICS:
(A) LENGTH: 1206 base pairs
(B) TYPE: nucleic acid
(C) STRANDEDNESS: single
(D) TOPOLOGY: linear
(ii) MOLECULE TYPE: cDNA

(xi) SEQUENCE DESCRIPTION: SEQ ID NO:4:

ATGAACAACT TGCTGTGCTG CGCGCTCGTG TTTCTGGACA TCTCCATTAA GTGGACCACC 60
CAGGAAACGT TTCCTCCAAA GTACCTTCAT TATGACGAAG AAACCTCTCA TCAGCTGTTG 120
TGTGACAAAT GTCCTCCTGG TACCTACCTA AAACAACACT GTACAGCAAA GTGGAAGACC 180
GTGTGCGCCC CTTGCCCTGA CCACTACTAC ACAGACAGCT GGCACACCAG TGACGAGTGT 240
CTATACTGCA GCCCCGTGTG CAAGGAGCTG CAGTACGTCA AGCAGGAGTG CAATCGCACC 300
CACAACCGCG TGTGCGAATG CAAGGAAGGG CGCTACCTTG AGATAGAGTT CTGCTTGAAA 360
CATAGGAGCT GCCCTCCTGG ATTTGGAGTG GTGCAAGCTG GAACCCCAGA GCGAAATACA 420
GTTTGCAAAA GATGTCCAGA TGGGTTCTTC TCAAATGAGA CGTCATCTAA AGCACCCTGT 480
AGAAAACACA CAAATTGCAG TGTCTTTGGT CTCCTGCTAA CTCAGAAAGG AAATGCAACA 540
CACGACAACA TATGTTCCGG AAACAGTGAA TCAACTCAAA AATGTGGAAT AGATGTTACC 600
CTGTGTGAGG AGGCATTCTT CAGGTTTGCT GTTCCTACAA AGTTTACGCC TAACTGGCTT 660
AGTGTCTTGG TAGACAATTT GCCTGGCACC AAAGTAAACG CAGAGAGTGT AGAGAGGATA 720
AAACGGCAAC ACAGCTCACA AGAACAGACT TTCCAGCTGC TGAAGTTATG GAAACATCAA 780
AACAAAGACC AAGATATAGT CAAGAAGATC ATCCAAGATA TTGACCTCTG TGAAAACAGC 840
GTGCAGCGGC ACATTGGACA TGCTAACCTC ACCTTCGAGC AGCTTCGTAG CTTGATGGAA 900
AGCTTACCGG GAAAGAAAGT GGGAGCAGAA GACATTGAAA AAACAATAAA GGCATGCAAA 960
CCCAGTGACC AGATCCTGAA GCTGCTCAGT TTGTGGCGAA TAAAAAATGG CGACCAAGAC 1020
ACCTTGAAGG GCCTAATGCA CGCACTAAAG CACTCAAAGA CGTACCACTT TCCCAAAACT 1080
GTCACTCAGA GTCTAAAGAA GACCATCAGG TTCCTTCACA GCTTCACAAT GTACAAATTG 1140
TATCAGAAGT TATTTTTAGA AATGATAGGT AACCAGGTCC AATCAGTAAA AATAAGCTGC 1200
TTATAA 1206

## Description

### FIELD OF TECHNOLOGY

The present invention relates to a novel DNA and a process for preparing a protein which possesses an activity to inhibit osteoclast differentiation and/or maturation (hereinafter called osteoclastogenesis-inhibitory activity) by a genetic engineering technique using the DNA. More particularly, the present invention relates to a genomic DNA encoding a protein OCIF which possesses an osteoclastogenesis-inhibitory activity and a process for preparing said protein by a genetic engineering technique using the genomic DNA.

### BACKGROUND OF THE INVENTION

Human bones are constantly repeating a process of resorption and formation. Osteoblasts controlling formation of bones and osteoclasts controlling resorption of bones take major roles in this process. Osteoporosis is a typical disease caused by abnormal metabolism of bones. This disease is caused when bone resorption by osteoclasts exceeds bone formation by osteoblasts. Although the mechanism of this disease is still to be elucidated completely, the disease causes the bones to ache, makes the bones fragile, and may results in fracturing of the bones. As the population of the aged increases, this disease results in an increase in bedridden aged people which becomes a social problem. Urgent development of a therapeutic agent for this disease is strongly desired. Disease due to a decrease in bone mass is expected to be treated by controlling bone resorption, accelerating bone formation, or improving balance between bone resorption and formation.

Osteogenesis is expected to increase by accelerating proliferation, differentiation, or activation of the cells controlling bone formation, or by controlling proliferation , differentiation, or activation of the cells involved in bone resorption. In recent years, strong interest has been directed to physiologically active proteins (cytokines) exhibiting such activities as described above, and energetic research is ongoing on this subject. The cytokines which have been reported to accelerate proliferation or differentiation of osteoblasts include the proteins of fibroblast growth factor family (FGF: Rodan S. B. et al., Endocrinology vol. 121, p l917, 1987), insulin-like growth factor I (IGF-I: Hock J. M. et al., Endocrinology vol. 122, p 254, 1988), insulin growth factor II (IGF-II: McCarthy T. et al., Endocrinology vol. 124, p 301, 1989), Activin A (Centrella M. et al., Mol. Cell. Biol., vol. 11, p 250, 1991), transforming growth factor-β, (Noda M., The Bone, vol. 2, p 29, 1988), Vasculotropin (Varonique M. et al., Biochem. Biophys. Res. Commun., vol. 199, p 380, 1994), and the protein of heterotopic bone formation factor family (bone morphogenic protein; BMP: BMP-2; Yanaguchi A. et al., J. Cell Biol. vol. 113, p 682, 1991, OP-1; Sampath T. K. et al., J. Biol. Chem. vol. 267, p 20532. 1992, and Knutsen R. et al., Biochem. Biophys. Res. Commun. vol. 194, P 1352, 1993).

On the other hand, as the cytokines which suppress differentiation and/or maturation of osteoclasts, transforming growth factor-β (Chenu C, et. al., Proc. Natl. Acad. Sci. USA, vol. 85, p 5683, 1988), interleukin-4 (Kasano K. et al., Bone-Miner., vol. 21, p 179, 1993), and the like have been reported. Further, as the cytokines which suppress bone resorption by osteoclast, calcitonin (Bone-Miner., vol. 17, p 347, 1992 ), macrophage colony stimulating factor (Hattersley G. et al., J. Cell. Physiol. vol. 137, p 199. 1988), interleukin-4 (Watanabe, K. et al., Biochem. Biophys. Res. Commun. vol. 172. P 1035, 1990), and interferon-γ (Gowen M. et al., J. Bone Miner. Res., vol. l, p 46.9, 1986) have been reported.

These cytokines are expected to be used as agents for treating diseases accompanying bone loss by accelerating bone formation or suppressing of bone resorption. Clinical tests are being undertaken to verify the effect of improving bone metabolism of some cytokines such as insulin-like growth factor-I and the heterotopic bone formation factor family. In addition, calcitonin is already commercially available as a therapeutic agent for osteoporosis and a pain relief agent. At present, drugs for clinically treating bone diseases or shortening the period of treatment of bone diseases include activated vitamin D₃, calcitonin and its derivatives, and hormone preparations such as estradiol agent, ipriflavon or calcium preparations. These agents are not necessarily satisfactory in terms of the efficacy and therapeutic results. Development of a novel therapeutic agent which can be used in place of these agents is strongly desired.

In view of this situation, the present inventors have undertaken extensive studies. As a result, the present inventors had found protein OCIF exhibiting an osteoclastogenesis-inhibitory activity in a culture broth of human embryonic lung fibroblast IMR-90 (ATCC Deposition No. CCL186), and filed a patent application (PCT/JP96/00374). The present inventors have conducted further studies relating to the origin of this protein OCIF exhibiting the osteoclastogenesis-inhibitory activity. The studies have matured into determination of the sequence of a genomic DNA encoding the human origin OCIF. Accordingly, an object of the present invention is to provide a genomic DNA encoding protein OCIF exhibiting osteoclastogenesis-inhibitory activity and a process for preparing this protein by a genetic engineering technique using the genomic DNA.

### DISCLOSURE OF THE INVENTION

Specifically, the present invention relates to a genomic DNA encoding protein OCIF exhibiting osteoclastogenesis-inhibitory activity and a process for preparing this protein by a genetic engineering technique using the genomic DNA. The DNA of the present invention includes the nucleotide sequences No. 1 and No. 2 in the Sequence Table attached hereto.

Moreover, the present invention relates to a process for preparing a protein, comprising inserting a DNA including the nucleotide sequences of the sequences No. 1 and No. 2 in the Sequence Table into an expression vector, producing a vector capable of expressing a protein having the following physicochemical characteristics and exhibiting the activity of inhibiting differentiation and/or maturation of osteoclasts, and producing this protein by a genetic engineering technique,
(a) molecular weight (SDS-PAGE):
   (i) Under reducing conditions: about 60 kD,
   (ii) Under non-reducing conditions: about 60 kD and about 120 kD;
(b) amino acid sequence:
   includes an amino acid sequence of the Sequence ID No. 3 of the Sequence Table,
(c) affinity:
   exhibits affinity to a cation exchanger and heparin, and
(d) thermal stability:
   (i) the osteoclast differentiation and/or maturation inhibitory activity is reduced when treated with heat at 70°C for 10 minutes or at 56°C for 30 minutes,
   (ii) the osteoclast differentiation and/or maturation inhibitory activity is lost when treated with heat at 90°C for 10 minutes.

The protein obtained by expressing the gene of the present invention exhibits an osteoclastogenesis-inhibitory activity. This protein is effective as an agent for the treatment and improvement of diseases involving decrease in the amount of bone such as osteoporosis, diseases relating to bone metabolism abnormality such as rheumatism, degenerative joint disease, or multiple myeloma, and is useful as an antigen to establish an immunological diagnosis of such diseases.

### BRIEF DESCRIPTION OF THE DRAWINGS

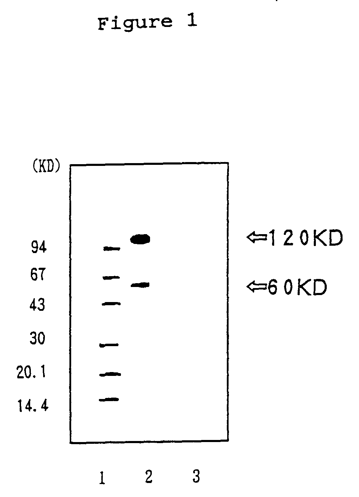

Figure 1 shows a result of Western Blotting analysis of the protein obtained by causing genomic DNA of the present invention to express a protein in Example 4 (iii), wherein lane 1 indicates a marker, lane 2 indicates the culture broth of COS7 cells in which a vector pWESRαOCIF (Example 4 (iii))has been transfected, and lane 3 is the culture broth of COS7 cell in which a vector pWESRα(control) has been transfected.

### BEST MODE FOR CARRYING OUT THE INVENTION

The genomic DNA encoding the protein OCIF which exhibits osteoclastogenesis-inhibitory activity in the present invention can be obtained by preparing a cosmid library using a human placenta genomic DNA and a cosmid vector and by screening this library using DNA fragments which are prepared based on the OCIF cDNA as a probe. The thus-obtained genomic DNA is inserted into a suitable expression vector to prepare an OCIF expression cosmid. A recombinant type OCIF can be obtained by transfecting the genomic DNA into a host organism such as various types of cells or microorganism strains and causing the DNA to express a protein by a conventional method. The resultant protein exhibiting osteoclastogenesis-inhibitory activity (an osteoclastogenesis-inhibitory factor) is useful as an agent for the treatment and improvement of diseases involving a decrease in bone mass such as osteoporosis and other diseases relating to bone metabolism abnormality and also as an antigen to prepare antibodies for establishing immunological diagnosis of such diseases. The protein of the present invention can be prepared as a drug composition for oral or non-oral administration. Specifically, the drug composition of the present invention containing the protein which is an osteoclastogenesis-inhibitory factor as an active ingredient can be safely administered to humans and animals. As the form of drug composition, a composition for injection, composition for intravenous drip, suppository, nasal agent, sublingual agent, percutaneous absorption agent, and the like are given. In the case of the composition for injection, such a composition is a mixture of a pharmacologically effective amount of osteoclastogenesis-inhibitory factor of the present invention and a pharmaceutically acceptable carrier. The composition may further comprise amino acids, saccharides, cellulose derivatives, and other excipients and/or activation agents, including other organic compounds and inorganic compounds which are commonly added to a composition for injection. When an injection preparation is prepared using the osteoclastogenesis-inhibitory factor of the present invention and these excipients and activation agents, a pH adjuster, buffering agent, stabilizer, solubilizing agent, and the like may be added if necessary to prepare various types of injection agents.

The present invention will now be described in more detail by way of examples which are given for the purpose of illustration and not intended to be limiting of the present invention.

### Example 1

### 〈Preparation of a cosmid library〉

A cosmid library was prepared using human placenta genomic DNA (Clonetech; Cat. No. 6550-2) and pWE15 cosmid vector (Stratagene). The experiment was carried out following principally the protocol attached to the pWE15 cosmid vector kit of Stratagene Company, provided Molecular Cloning: A Laboratory Mannual (Cold Spring Harbor Laboratory (1989)) was referred to for common procedures for handling DNA, E. coli, and pharge.

### (i) Preparation of restrictive enzymolysate of human-genomic DNA

Human placenta genomic DNA dissolved in 750 µl of a solution containing 10 mM Tris-HCl, 10 mM MgCl₂, and 100 mM NaCl was added to four 1.5 ml Eppendorf tubes (tube A, B, C, and D) in the amount of 100 µg each. Restriction enzyme MboI was added to these tubes in the amounts of 0.2 unit for tube A, 0.4 unit for tube B, 0.6 unit for tube C, and 0.8 unit for tube D, and DNA was digested for 1 hour. Then, EDTA in the amount to make a 20 mM concentration was added to each tube to terminate the reaction, followed by extraction with phenol/chloroform (1:1). A two-fold amount of ethanol was added to the aqueous layer to precipitate DNA. DNA was collected by centrifugation, washed with 70% ethanol, and DNA in each tube was dissolved in 100 µl of TE (10 mM HCl (pH 8.0) + 1 mM EDTA buffer solution, hereinafter called TE). DNA in four tubes was combined in one tube and incubated for 10 minutes at 68°C. After cooling to room temperature, the mixture was overlayed onto a 10%-40 % linear sucrose gradient which was prepared in a buffer containing 20 mM Tris-HC1 (pH 8.0), 5 mM EDTA, and 1 mM NaC1 in an centrifugal tube (38 ml). The tube was centrifuged at 26,000 rpm for 24 hours at 20°C using a rotor SRP28SA manufactured by Hitachi, Ltd. and 0.4 ml fractions of the sucrose gradient was collected using a fraction collector. A portion of each fraction was subjected to 0.4% agarose electrophoresis to confirm the size of DNA. Fractions containing DNA with a length of 30 kb (kilo base pair) to 40 kb were thus combined. The DNA solution was diluted with TE to make a sucrose concentration to 10% or less and 2.5-fold volumes of ethanol was added to precipitate DNA. DNA was dissolved in TE and stored at 4°C.

### (ii) Preparation of cosmid vector

The pWE15 cosmid vector obtained from Stratagene Company was completely digested with restriction enzyme BamHI according to the protocol attached to the cosmid vector kit. DNA collected by ethanol precipitation was dissolved in TE to a concentration of 1 mg/m1. Phosphoric acid at the 5'-end of this DNA was removed using calf small intestine alkaline phosphatase, and DNA was collected by phenol extraction and ethanol precipitation. The DNA was dissolved in TE to a concentration of 1 mg/ml.

### (iii) Ligation of genomic DNA to vector and in vitro packaging

1.5 micrograms of genomic DNA fractionated according to size and 3 µg of pWE15 cosmid vector which was digested with restriction enzyme BamHI were ligated in 20 µl of a reaction solution using Ready-To-Go T4DNA ligase of Pharmacia Company. The ligated DNA was packaged in vitro using Gigapack™ II packaging extract (Stratagene) according to the protocol. After the packaging reaction, a portion of the reaction mixture was diluted stepwise with an SM buffer solution and mixed with E. coli XL1-Blue MR (Stratagene) which was suspended in 10 mM MgC1₂ to cause pharge to infect, and plated onto LB agar plates containing 50 µg/ml of ampicillin. The number of colonies produced was counted. The number of colonies per 1 µl of packaging reaction was calculated based on this result.

### (iv) Preparation of a cosmid library

The packaging reaction solution thus prepared was mixed with E. coli XL1-Blue MR and the mixture was plated onto agarose plates containing ampicillin so as to produce 50,000 colonies per agarose plate having a 15 cm of diameter. After incubating the plate overnight at 37°C, an LB culture medium was added in the amount of 3 ml per plate to suspend and collect colonies of E. coli. Each agarose plate was again washed with 3 ml of the LB culture medium and the washing was combined with the original suspension of E. coli. The E. coli collected from all agarose plates was placed in a centrifugal tube, glycerol was added to a concentration of 20%, and ampicillin was further added to make a final concentration of 50 µg/m1. A portion of the E. coli suspension was removed and the remainder was stored at -80°C. The removed E. coli was diluted stepwise and plated onto an agar plates to count the number of colonies per 1 ml of suspension.

### Example 2

### 〈Screening of cosmid library and purification of colony〉

A nitrocellulose filter (Millipore) with a diameter of 14.2 cm was placed on each LB agarose plate with a diameter of 15 cm which contained 50 µg/m1 of ampicillin. The cosmid library was plated onto the plates so as to produce 50,000 colonies of E. coli per plate, followed by incubation overnight at 37°C. E. coli on the nitrocellulose filter was transferred to another nitrocellulose filter according to a conventional method to obtain two replica filters. According to the protocol attached to the cosmid vector kit, cosmid DNA in the E. coli on the replica filters was denatured with an alkali, neutralized, and immobilized on the nitrocellulose filter using a Stratalinker (Stratagene). The filters were heated for two hours at 80°C in a vacuum oven. The nitrocellulose filters thus obtained were hybridized using two kinds of DNA produced, respectively, from 5'-end and 3'-end of human OCIF cDNA as probes. Namely, a plasmid was purified from E. coli pKB/OIF10 (deposited at The Ministry of International Trade and Industry, the Agency of Industrial Science and Technology, Biotechnology Laboratory, Deposition No. FERM BP-5267) containing OCIF cDNA. The plasmid containing OCIF cDNA was digested with restriction enzymes KpnI and EcoRI. Fragments thus obtained was separated using agarose gel electrophoresis. KpnI/EcoRI fragment with a length of 0.2 kb was purified using a QlAEX II gel extraction kit (Qiagen). This DNA was labeled with ³²p using the Megaprime DNA Labeling System (Amasham) (5'-DNA probe). Apart from this, a BamHI/EcoRV fragment with a length of 0.2 kb which was produced from the above plasmid by digestion with restriction enzymes BamHI and EcoRV was purified and labeled with ³²p (3'-DNA probe). One of the replica filters described above was hybridized with the 5'-DNA probe and the other with the 3'-DNA probe. Hybridization and washing of the filters were carried out according to the protocol attached to the cosmid vector kit. Autoradiography detected several positive signals with each probe. One colony which gave positive signals with both probe was identified. The colony on the agar plate, which corresponding to the signal on the autoradiogram was isolated and purified. A cosmid was prepared from the purified colony by a conventional method. This cosmid was named pWEOCIF. The size of human genomic DNA contained in this cosmid was about 38 kb.

### Example 3

### 〈Determination of the nucleotide sequence of human OCIF genomic DNA〉

### (i) Subcloning of OCIF genomic DNA

Cosmid pWEOCIF was digested with restriction enzyme EcoRI. After the separation of the DNA fragments thus produced by electrophoresis using a 0.7% agarose gel, the DNA fragments were transferred to a nylon membrane (Hybond -N, Amasham) by the Southern blot technique and immobilized on the nylon membrane using Stratalinker (Stratagene). On the other hand, plasmid pBKOCIF was digested with restriction enzyme EcoRI and a 1.6 kb fragment containing human OCIF cDNA was isolated by agarose gel electrophoresis. The fragment was labeled with ³²P using the Megaprime DNA labeling system (Amasham).

Hybridization of the nylon membranes described above with the ³²P-labeled 1.6-kb OCIF cDNA was performed according to a conventional method detected that DNA fragments with a size of 6 kb, 4 kb, 3.6 kb, and 2.6 kb. These fragments hybridized with the human OCIF cDNA were isolated using agarose gel electrophoresis and individually subcloned into an EcoRI site of pBluescript II SK + vector (Strategene) by a conventional method. The resulting plasmids were respectively named pBSE 6, pBSE 4, pBSE 3.6, and PBSE 2.6.

### (ii) Determination of the nucleotide sequence

The nucleotide sequence of human OCIF genomic DNA which was subcloned into the plasmid was determined using the ABI Dideoxy Terminator Cycle Sequencing Ready Reaction kit (Perkin Elmer) and the 373 Sequencing System (Applied Biosystems). The primer used for the determination of the nucleotide sequence was synthesized based on the nucleotide sequence of human OCIF cDNA (Sequence ID No. 4 in the Sequence Table). The nucleotide sequences thus determined are given as the Sequences No. 1 and No. 2 in the Sequence Table. The Sequence ID No. 1 includes the first exon of the OCIF gene and the Sequence ID No. 2 includes the second, third, fourth, and fifth exons. A stretch of about 17 kb is present between the first and second exons.

### Example 4

### 〈Production of recombinant OCIF using COS-7 cells〉

### (i) Preparation of OCIF genomic DNA expression cosmid

To express OCIF genomic DNA in animal cells, an expression unit of expression plasmid pcDL-SRα296 (Molecular and Cellar Biology, vol. 8, P466-472, 1988) was inserted into cosmid vector pWE15 (Stratagene). First of all, the expression plasmid pcDL-SRα296 was digested with a restriction enzyme Sal I to cut out expression unit with a length of about 1.7 kb which includes an SRαpromotor, SV40 later splice signal, poly (A) addition signal, and so on. The digestion products were separated by agarose electrophoresis and the 1.7-kb fragment was purified using the QIAEX II gel extraction kit (Qiagen). On the other hand, cosmid vector pWE15 was digested with a restriction enzyme EcoRI and fragments were separated using agarose gel electrophoresis. pWE15 DNA of 8.2 kb long was purified using the QIAEX II gel extraction kit (Qiagen). The ends of these two DNA fragments were bluntled using a DNA blunting kit (Takara Shuzo), ligated using a DNA ligation kit (Takara Shuzo), and transferred into E. coli DH5α (Gibco BRL). The resultant transformant was grown and the expression cosmid pWESRα containing an expression unit was purified using a Qiagen column (Qiagen).

The cosmid pWE OCIF containing the OCIF genomic DNA with a length of about 38 kb obtained in (i) above was digested with a restriction enzyme NotI to cut out the OCIF genomic DNA of about 38 kb. After separation by agarose gel electrophoresis, the DNA was purified using the QIAEX II gel extraction kit (Qiagen). On the other hand, the expression cosmid pWESRα was digested with a restriction enzyme EcoRI and the digestion product was extracted with phenol and chloroform, ethanol-precipitated, and dissolved in TE.

pWESRα digested with a restriction enzyme EcoRI and an EcoRI-XmnI-NotI adapter (#1105, #1156 New England Biolaboratory Co.) were ligated using T4 DNA ligase (Takara Shuzo Co., Ltd.). After removal of the free adapter by agarose gel electrophoresis, the product was purified using QIAEX gel extraction kit (Qiagen). The OCIF genomic DNA with a length of about 37 kb which was derived from the digestion with restriction enzyme NotI and the pWESRα to which the adapter was attached were ligated using T4 DNA ligase (Takara Shuzo). The DNA was packaged in vitro using the Gigapack packaging extract (Stratagene) and infected with E. coli XL1-Blue MR (Stratagene). The resultant transformant was grown and the expression cosmid pWESRαOCIF which contained OCIF genomic DNA was inserted was purified using a Qiagen column (Qiagen). The OCIF expression cosmid pWESRαOCIF was ethanol-precipitated and dissolved in sterile distilled water and used in the following analysis.

### (ii) Transient expression of OCIF genomic DNA and measurement of OCIF activity

A recombinant OCIF was expressed as described below using the OCIF expression cosmid pWESRαOCIF obtained in (i) above and its activity was measured. COS-7 (8x10⁵cells/well) cells (Riken Cell Bank, RCB0539) were planted in a 6-well plate using DMEM culture medium (Gibco BRL) containing 10% fetal bovine serum (Gibco BRL). On the following day, the culture medium was removed and cells were washed with serum-free DMEM culture medium. The OCIF expression cosmid pWESRαOCIF which had been diluted with OPTI-MEM culture medium (Gibco BRL) was mixed with lipophectamine and the mixture was added to the cells in each well according to the attached protocol. The expression cosmid pWESRα was added to the cells in the same manner as a control. The amount of the cosmid DNA and Lipophectamine was respectively 3 µg and 12 µl. After 24 hours, the culture medium was removed and 1.5 m1 of fresh EX-CELL 301 culture medium (JRH Bioscience) was added to each well. The culture medium was recovered after 48 hours and used as a sample for the measurement of OCIF activity. The measurement of OCIF activity was carried out according to the method described by Kumegawa, M. et al. (Protein, Nucleic Acid, and Enzyme, Vo1. 34, p 999 (1989)) and the method of TAKAHASHI, N. et al. (Endocrihology vol. 122, p 1373 (1988)). The osteoclast formation in the presence of activated vitamin D₃ from bone marrow cells isolated from mice aged about 17 days was evaluated by the induction of tartaric acid resistant acidic phosphatase activity. The inihibition of the acid phosphatase was measured and used as the activity of the protein which possesses osteoclastogenesis-inhibitory activity (OCIF). Namely, 100 µl/well of a OCIF sample which was diluted with α-MEM culture medium (Gibco BRL) containing 2x10⁻⁸ M activated vitamin D₃ and 10% fetal bovine serum was added to each well of a 96 well micro plate. Then, 3x10⁵ bone marrow cells isolated from mice (about 17-days old) suspended in 100 µl of α-MEM culture medium containing 10% fetal bovine serum were added to each well of the 96 well micro plate and cultured for a week at 37°C and 100% humidity under 5% CO₂ atmosphere. On days 3 and 5, 160 µl of the conditioned medium was removed from each well, and 160 µl of a sample which was diluted with α-MEM culture medium containing 1x10⁻⁸ M activated vitamin D₃ and 10% fetal bovine serum was added. After 7 days from the start of culturing, the cells were washed with a phosphate buffered saline and fixed with a ethanol/acetone (1:1) solution for one minute at room temperature. The osteoclast formation was detected by staining the cells using an acidic phosphatase activity measurement kit (Acid Phosphatase, Leucocyte, Cat.No. 387-A, Sigma Company). A decrease in the number of cells positive to acidic phosphatase activity in the presence of tartaric acid was taken as the OCIF activity. The results are shown in Table 1, which indicates that the conditioned medium exhibits the similar activity to natural type OCIF obtained from the IMR-90 culture medium and recombinant OCIF produced by CHO cells.

**TABLE 1**

| Activity of OCIF expressed by COS-7 cells in the conditioned medium | | | | | | |
|---|---|---|---|---|---|---|
| Dilution | 1/10 | 1/20 | 1/40 | 1/80 | 1/160 | 1/320 |
| OCIF genomic DNA introduced | ++ | ++ | ++ | ++ | + | - |
| Vector introduced | - | - | - | - | - | - |
| Untreated | - | - | - | - | - | - |
| "++" indicates an activity inhibiting 80% or more of osteoclast formation, "+" indicates an activity inhibiting 30-80% of osteoclast formation, and "-" indicates that no inhibition of osteoclast formation is observed. | | | | | | |

### (iii) Identification of the product by Western Blotting

A buffer solution (10 µl) for SDS-PAGE (0.5 M Tris-HC1, 20% glycerol, 4% SDS, 20 µg/m1 bromophenol blue, pH 6.8) was added to 10 µ1 of the sample for the measurement of OCIF activity prepared in (ii) above. After boiling for 3 minutes at 100°C, the mixture was subjected to 10% SDS polyacrylamide electrophoresis under non-reducing conditions. The proteins were transferred from the gel to a PVDF membrane (ProBlott, Perkin Elmer) using semi-dry blotting apparatus (Biorad). The membrane was blocked and incubated for 2 hours at 37°C together with a horseradish peroxidase-labeled anti-OCIF antibody obtained by labeling the previously obtained OCIF protein with horseradish peroxidase according to a conventional method. After washing, the protein which has bound the anti-OCIF antibody was detected using the ECL system (Amasham). As shown in Figure 1, two bands, one with a molecular weight of about 120 kilo dalton and the other 60 kilo dalton, were detected in the supernatant obtained from the culture broth of COS-7 cells in which pWESRαOCIF was transfected. On the other hand, these two bands with a molecular weight of about 120 kilo dalton and 60 kilo dalton were not detected in the supernatant obtained from the culture broth of COS-7 cells in which pWESRαvector was transfected, confirming that the protein obtained was OCIF.

### INDUSTRIAL APPLICABILITY

The present invention provides a genomic DNA encoding a protein OCIF which possesses an osteoclastogenesis-inhibitory activity and a process for preparing this protein by a genetic engineering technique using the genomic DNA. The protein obtained by expressing the gene of the present invention exhibits an osteoclastogenesis-inhibitory activity and is useful as an agent for the treatment and improvement of diseases involving a decrease in the amount of bone such as osteoporosis, other diseases resulting from bone metabolism abnormality such as rheumatism or degenerative joint disease, and multiple myeloma. The protein is further useful as an antigen to establish antibodies useful for an immunological diagnosis of such diseases.

### NOTE ON MICROORGANISM

- Depositing Organization:: The Ministry of International Trade and Industry, National Institute of Bioscience and Human Technology, Agency of Industrial Science and Technology
- Address:: 1-3, Higashi-1-Chome, Tsukuba-shi, Ibaraki-ken, Japan
- Date of Deposition:: June 21, 1995 (originally deposited on June 21, 1995 and transferred to the international deposition according to the Budapest Treaty on October 25, 1995)
Accession No. FERM BP-5267

TABLE OF SEQUENCES

Sequence number: 1

Length of sequence: 1316

Sequence Type: nucleic acid

Strandedness: double

Topology: linear

Molecular type: genomic DNA (human OCIF genomic DNA-1)

Sequence:
CTGGAGACAT ATAACTTGAA CACTTGGCCC TGATGGGGAA GCAGCTCTGC AGGGACTTTT 60
TCAGCCATCT GTAAACAATT TCAGTGGCAA CCCGCGAACT GTAATCCATG AATGGGACCA 120
CACTTTACAA GTCATCAAGT CTAACTTCTA GACCAGGGAA TTAATGGGGG AGACAGCGAA 180
CCCTAGAGCA AAGTGCCAAA CTTCTGTCGA TAGCTTGAGG CTAGTGGAAA GACCTCGAGG 240
AGGCTACTCC AGAAGTTCAG CGCGTAGGAA GCTCCGATAC CAATAGCCCT TTGATGATGG 300
TGGGGTTGGT GAAGGGAACA GTGCTCCGCA AGGTTATCCC TGCCCCAGGC AGTCCAATTT 360
TCACTCTGCA GATTCTCTCT GGCTCTAACT ACCCCAGATA ACAAGGAGTG AATGCAGAAT 420
AGCACGGGCT TTAGGGCCAA TCAGACATTA GTTAGAAAAA TTCCTACTAC ATGGTTTATG 480
TAAACTTGAA GATGAATGAT TGCGAACTCC CCGAAAAGGG CTCAGACAAT GCCATGCATA 540
AAGAGGGGCC CTGTAATTTG AGGTTTCAGA ACCCGAAGTG AAGGGGTCAG GCAGCCGGGT 600
ACGGCGGAAA CTCACAGCTT TCGCCCAGCG AGAGGACAAA GGTCTGGGAC ACACTCCAAC 660
TGCGTCCGGA TCTTGGCTGG ATCGGACTCT CAGGGTGGAG GAGACACAAG CACAGCAGCT 720
GCCCAGCGTG TGCCCAGCCC TCCCACCGCT GGTCCCGGCT GCCAGGAGGC TGGCCGCTGG 780
CGGGAAGGGG CCGGGAAACC TCAGAGCCCC GCGGAGACAG CAGCCGCCTT GTTCCTCAGC 840
CCGGTGGCTT TTTTTTCCCC TGCTCTCCCA GGGGACAGAC ACCACCGCCC CACCCCTCAC 900
GCCCCACCTC CCTGGGGGAT CCTTTCCGCC CCAGCCCTGA AAGCGTTAAT CCTGGAGCTT 960
TCTGCACACC CCCCGACCGC TCCCGCCCAA GCTTCCTAAA AAAGAAAGGT GCAAAGTTTG 1020
GTCCAGGATA GAAAAATGAC TGATCAAAGG CAGGCGATAC TTCCTGTTGC CGGGACGCTA 1080
TATATAACGT GATGAGCGCA CGGGCTGCGG AGACGCACCG GAGCGCTCGC CCAGCCGCCG 1140

CCTCCAAGCC CCTGAGGTTT CCGGGGACCA CA ATG AAC AAG TTG CTG TGC TGC 1193
Met Asn Lys Leu Leu Cys Cys
-20 -15

GCG CTC GTG GTAAGTCCCT GGGCCAGCCG ACGGGTGCCC GGCGCCTGGG 1242
Ala Leu Val

GAGGCTGCTG CCACCTGGTC TCCCAACCTC CCAGCGGACC GGCGGGGAGA AGGCTCCACT 1302
CGCTCCCTCC CAGG 1316

Sequence number: 2
Length of sequence: 9898
Sequence Type: nucleic acid
Strandedness: double
Topology: linear
Molecular type: genomic DNA (human OCIF genomic DNA-2)

Sequence:
GCTTACTTTG TGCCAAATCT CATTAGGCTT AAGGTAATAC AGGACTTTGA GTCAAATGAT 60
ACTGTTGCAC ATAAGAACAA ACCTATTTTC ATGCTAAGAT GATGCCACTG TGTTCCTTTC 120
TCCTTCTAG TTT CTG GAC ATC TCC ATT AAG TGG ACC ACC CAG GAA ACG TTT 171
Phe Leu Asp Ile Ser Ile Lys Trp Thr Thr Gln Glu Thr Phe
-10 -5 1

CCT CCA AAG TAC CTT CAT TAT GAC GAA GAA ACC TCT CAT CAG CTG TTG 219
Pro Pro Lys Tyr Leu His Tyr Asp Glu Glu Thr Ser His Gln Leu Leu
                5                   10                  15

TGT GAC AAA TGT CCT CCT GGT ACC TAC CTA AAA CAA CAC TGT ACA GCA 267
Cys Asp Lys Cys Pro Pro Gly Thr Tyr Leu Lys Gln His Cys Thr Ala
20 25 30 35

AAG TGG AAG ACC GTG TGC GCC CCT TGC CCT GAC CAC TAC TAC ACA GAC 315
Lys Trp Lys Thr Val Cys Ala Pro Cys Pro Asp His Tyr Tyr Thr Asp
40 45 50

AGC TGG CAC ACC AGT GAC GAG TGT CTA TAC TGC AGC CCC GTG TGC AAG 363
Ser Trp His Thr Ser Asp Glu Cys Leu Tyr Cys Ser Pro Val Cys Lys
55 60 65

GAG CTG CAG TAC GTC AAG CAG GAG TGC AAT CGC ACC CAC AAC CGC GTG 411
Glu Leu Gln Tyr Val Lys Gln Glu Cys Asn Arg Thr His Asn Arg Val
70 75 80

TGC GAA TGC AAG GAA GGG CGC TAC CTT GAG ATA GAG TTC TGC TTG AAA 459
Cys Glu Cys Lys Glu Gly Arg Tyr Leu Glu Ile Glu Phe Cys Leu Lys
                85                  90                  95

CAT AGG AGC TGC CCT CCT GGA TTT GGA GTG GTG CAA GCT G GTACGTGTCA 509
His Arg Ser Cys Pro Pro Gly Phe Gly Val Val Gln Ala
100 105 110

ATGTGCAGCA AAATTAATTA GGATCATGCA AAGTCAGATA GTTGTGACAG TTTAGGAGAA 569

CACTTTTGTT CTGATGACAT TATAGGATAG CAAATTGCAA AGGTAATGAA ACCTGCCAGG 629
TAGGTACTAT GTGTCTGGAG TGCTTCCAAA GGACCATTGC TCAGAGGAAT ACTTTGCCAC 689
TACAGGGCAA TTTAATGACA AATCTCAAAT GCAGCAAATT ATTCTCTCAT GAGATGCATG 749
ATGGTTTTTT TTTTTTTTTT TAAAGAAACA AACTCAAGTT GCACTATTGA TAGTTGATCT 809
ATACCTCTAT ATTTCACTTC AGCATGGACA CCTTCAAACT GCAGCACTTT TTGACAAACA 869
TCAGAAATGT TAATTTATAC CAAGAGAGTA ATTATGCTCA TATTAATGAG ACTCTGGAGT 929
GCTAACAATA AGCAGTTATA ATTAATTATG TAAAAAATGA GAATGGTCAG GGGAATTGCA 989
TTTCATTATT AAAAACAAGG CTAGTTCTTC CTTTAGCATG GGAGCTGAGT GTTTGGGAGG 1049
GTAAGGACTA TAGCAGAATC TCTTCAATGA GCTTATTCTT TATCTTAGAC AAAACAGATT 1109
GTCAAGCCAA GAGCAAGCAC TTGCCTATAA ACCAAGTGCT TTCTCTTTTG CATTTTGAAC 1169
AGCATTGGTC AGGGCTCATG TGTATTGAAT CTTTTAAACC AGTAACCCAC GTTTTTTTTC 1229
TGCCACATTT GCGAAGCTTC AGTGCAGCCT ATAACTTTTC ATAGCTTGAG AAAATTAAGA 1289
GTATCCACTT ACTTAGATGG AAGAAGTAAT CAGTATAGAT TCTGATGACT CAGTTTGAAG 1349
CAGTGTTTCT CAACTGAAGC CCTGCTGATA TTTTAAGAAA TATCTGGATT CCTAGGCTGG 1409
ACTCCTTTTT GTGGGCAGCT GTCCTGCGCA TTGTAGAATT TTGGCAGCAC CCCTGGACTC 1469
TAGCCACTAG ATACCAATAG CAGTCCTTCC CCCATGTGAC AGCCAAAAAT GTCTTCAGAC 1529
ACTGTCAAAT GTCGCCAGGT GGCAAAATCA CTCCTGGTTG AGAACAGGGT CATCAATGCT 1589
AAGTATCTGT AACTATTTTA ACTCTCAAAA CTTGTGATAT ACAAAGTCTA AATTATTAGA 1649
CGACCAATAC TTTAGGTTTA AAGGCATACA AATGAAACAT TCAAAAATCA AAATCTATTC 1709
TGTTTCTCAA ATAGTGAATC TTATAAAATT AATCACAGAA GATGCAAATT GCATCAGAGT 1769
CCCTTAAAAT TCCTCTTCGT ATGAGTATTT GAGGGAGGAA TTGGTGATAG TTCCTACTTT 1829
CTATTGGATG GTACTTTGAG ACTCAAAAGC TAAGCTAAGT TGTGTGTGTG TCAGGGTGCG 1889
GGGTGTGGAA TCCCATCAGA TAAAAGCAAA TCCATGTAAT TCATTCAGTA AGTTGTATAT 1949
GTAGAAAAAT GAAAAGTGGG CTATGCAGCT TGGAAACTAG AGAATTTTGA AAAATAATGC 2009
AAATCACAAG GATCTTTCTT AAATAAGTAA GAAAATCTGT TTGTAGAATG AAGCAAGCAG 2069
GCAGCCAGAA GACTCAGAAC AAAAGTACAC ATTTTACTCT GTGTACACTG GCAGCACAGT 2129

GGGATTTATT TACCTCTCCC TCCCTAAAAA CCCACACAGC GGTTCCTCTT GGGAAATAAG 2189
AGGTTTCCAG CCCAAAGAGA AGGAAAGACT ATGTGGTGTT ACTCTAAAAA GTATTTAATA 2249
ACCGTTTTGT TGTTGCTGTT GCTGTTTTGA AATCAGATTG TCTCCTCTCC ATATTTTATT 2309
TACTTCATTC TGTTAATTCC TGTGGAATTA CTTAGAGCAA GCATGGTGAA TTCTCAACTG 2369
TAAAGCCAAA TTTCTCCATC ATTATAATTT CACATTTTGC CTGGCAGGTT ATAATTTTTA 2429
TATTTCCACT GATAGTAATA AGGTAAAATC ATTACTTAGA TGGATAGATC TTTTTCATAA 2489
AAAGTACCAT CAGTTATAGA GGGAAGTCAT GTTCATGTTC AGGAAGGTCA TTAGATAAAG 2549
CTTCTGAATA TATTATGAAA CATTAGTTCT GTCATTCTTA GATTCTTTTT GTTAAATAAC 2609
TTTAAAAGCT AACTTACCTA AAAGAAATAT CTGACACATA TGAACTTCTC ATTAGGATGC 2669
AGGAGAAGAC CCAAGCCACA GATATGTATC TGAAGAATGA ACAAGATTCT TAGGCCCGGC 2729
ACGGTGGCTC ACATCTGTAA TCTCAAGAGT TTGAGAGGTC AAGGCGGGCA GATCACCTGA 2789
GGTCAGGAGT TCAAGACCAG CCTGGCCAAC ATGATGAAAC CCTGCCTCTA CTAAAAATAC 2849
AAAAATTAGC AGGGCATGGT GGTGCATGCC TGCAACCCTA GCTACTCAGG AGGCTGAGAC 2909
AGGAGAATCT CTTGAACCCT CGAGGCGGAG GTTGTGGTGA GCTGAGATCC CTCTACTGCA 2969
CTCCAGCCTG GGTGACAGAG ATGAGACTCC GTCCCTGCCG CCGCCCCCGC CTTCCCCCCC 3029
AAAAAGATTC TTCTTCATGC AGAACATACG GCAGTCAACA AAGGGAGACC TGGGTCCAGG 3089
TGTCCAAGTC ACTTATTTCG AGTAAATTAG CAATGAAAGA ATGCCATGGA ATCCCTGCCC 3149
AAATACCTCT GCTTATGATA TTGTAGAATT TGATATAGAG TTGTATCCCA TTTAAGGAGT 3209
AGGATGTAGT AGGAAAGTAC TAAAAACAAA CACACAAACA GAAAACCCTC TTTGCTTTGT 3269
AAGGTGGTTC CTAAGATAAT GTCAGTGCAA TGCTGGAAAT AATATTTAAT ATGTGAAGGT 3329
TTTAGGCTGT GTTTTCCCCT CCTGTTCTTT TTTTCTGCCA GCCCTTTGTC ATTTTTGCAG 3389
GTCAATGAAT CATGTAGAAA GAGACAGGAG ATGAAACTAG AACCAGTCCA TTTTGCCCCT 3449
TTTTTTATTT TCTGGTTTTG GTAAAAGATA CAATGAGGTA GGAGGTTGAG ATTTATAAAT 3509
GAAGTTTAAT AAGTTTCTGT AGCTTTGATT TTTCTCTTTC ATATTTGTTA TCTTGCATAA 3569
GCCAGAATTG GCCTGTAAAA TCTACATATG GATATTGAAG TCTAAATCTG TTCAACTAGC 3629
TTACACTAGA TGGAGATATT TTCATATTCA GATACACTGG AATGTATGAT CTAGCCATGC 3689

GTAATATAGT CAAGTGTTTG AAGGTATTTA TTTTTAATAG CGTCTTTAGT TGTGGACTGG 3749

TTCAAGTTTT TCTGCCAATG ATTTCTTCAA ATTTATCAAA TATTTTTCCA TCATGAAGTA 3809

AAATGCCCTT GCAGTCACCC TTCCTGAAGT TTGAACGACT CTGCTGTTTT AAACAGTTTA 3869

AGCAAATGGT ATATCATCTT CCGTTTACTA TGTAGCTTAA CTGCAGGCTT ACGCTTTTGA 3929

GTCAGCGGCC AACTTTATTG CCACCTTCAA AAGTTTATTA TAATGTTGTA AATTTTTACT 3989

TCTCAAGGTT AGCATACTTA GGAGTTGCTT CACAATTAGG ATTCAGGAAA GAAAGAACTT 4049

CAGTAGGAAC TGATTGGAAT TTAATGATGC AGCATTCAAT GGGTACTAAT TTCAAAGAAT 4109

GATATTACAG CAGACACACA GCAGTTATCT TGATTTTCTA GGAATAATTG TATGAAGAAT 4169

ATGGCTGACA ACACGGCCTT ACTGCCACTC AGCGGAGGCT GGACTAATGA ACACCCTACC 4229

CTTCTTTCCT TTCCTCTCAC ATTTCATGAG CGTTTTGTAG GTAACGAGAA AATTGACTTG 4289

CATTTGCATT ACAAGGAGGA GAAACTGGCA AAGGGGATGA TGGTGGAAGT TTTGTTCTGT 4349

CTAATGAAGT GAAAAATGAA AATGCTAGAG TTTTGTGCAA CATAATAGTA GCAGTAAAAA 4409

CCAAGTGAAA AGTCTTTCCA AAACTGTGTT AAGAGGGCAT CTGCTGGGAA ACGATTTGAG 4469

GAGAAGGTAC TAAATTGCTT GGTATTTTCC GTAG GA ACC CCA GAG CGA AAT ACA 4523

Gly Thr Pro Glu Arg Asn Thr

115

GTT TGC AAA AGA TGT CCA GAT GGG TTC TTC TCA AAT GAG ACG TCA TCT 4571

Val Cys Lys Arg Cys Pro Asp Gly Phe Phe Ser Asn Glu Thr Ser Ser

120 125 130 135

AAA GCA CCC TGT AGA AAA CAC ACA AAT TGC AGT GTC TTT GGT CTC CTG 4619

Lys Ala Pro Cys Arg Lys His Thr Asn Cys Ser Val Phe Gly Leu Leu

140 145 150

CTA ACT CAG AAA GGA AAT GCA ACA CAC GAC AAC ATA TGT TCC GGA AAC 4667

Leu Thr Gln Lys Gly Asn Ala Thr His Asp Asn Ile Cys Ser Gly Asn
155 160 165

AGT GAA TCA ACT CAA AAA TGT GGA ATA G GTAATTACAT TCCAAAATAC 4715
Ser Glu Ser Thr Gln Lys Cys Gly Ile
170 175

GTCTTTGTAC GATTTTGTAG TATCATCTCT CTCTCTGAGT TGAACACAAG GCCTCCAGCC 4775
ACATTCTTGG TCAAACTTAC ATTTTCCCTT TCTTGAATCT TAACCAGCTA AGGCTACTCT 4835
CGATGCATTA CTGCTAAAGC TACCACTCAG AATCTCTCAA AAACTCATCT TCTCACAGAT 4895
AACACCTCAA AGCTTGATTT TCTCTCCTTT CACACTGAAA TCAAATCTTG CCCATAGGCA 4955
AAGGGCAGTG TCAAGTTTGC CACTGAGATG AAATTAGGAG AGTCCAAACT GTAGAATTCA 5015
CGTTGTGTGT TATTACTTTC ACGAATGTCT GTATTATTAA CTAAAGTATA TATTGGCAAC 5075
TAAGAAGCAA AGTGATATAA ACATGATGAC AAATTAGGCC AGGCATGGTG GCTTACTCCT 5135
ATAATCCCAA CATTTTGGGG GGCCAAGGTA GGCAGATCAC TTGAGGTCAG GATTTCAAGA 5195
CCAGCCTGAC CAACATGGTG AAACCTTGTC TCTACTAAAA ATACAAAAAT TAGCTGGGCA 5255
TGGTAGCAGG CACTTCTAGT ACCAGCTACT CAGGGCTGAG GCAGGAGAAT CGCTTGAACC 5315
CAGGAGATGG AGGTTGCAGT GAGCTGAGAT TGTACCACTG CACTCCAGTC TGGGCAACAG 5375
AGCAAGATTT CATCACACAC ACACACACAC ACACACACAC ACACATTAGA AATGTGTACT 5435
TGGCTTTGTT ACCTATCGTA TTAGTGCATC TATTGCATGG AACTTCCAAG CTACTCTGGT 5495
TGTGTTAAGC TCTTCATTGG GTACAGGTCA CTAGTATTAA GTTCAGGTTA TTCGGATGCA 5555
TTCCACGGTA GTGATGACAA TTCATCAGGC TAGTGTGTGT GTTCACCTTG TCACTCCCAC 5615
CACTAGACTA ATCTCAGACC TTCACTCAAA GACACATTAC ACTAAAGATG ATTTGCTTTT 5675
TTGTGTTTAA TCAAGCAATG GTATAAACCA GCTTGACTCT CCCCAAACAG TTTTTCGTAC 5735
TACAAAGAAG TTTATGAAGC AGAGAAATGT GAATTGATAT ATATATGAGA TTCTAACCCA 5795
GTTCCAGCAT TGTTTCATTG TGTAATTGAA ATCATAGACA AGCCATTTTA GCCTTTGCTT 5855

TCTTATCTAA AAAAAAAAAA AAAAAAATGA AGGAAGGGGT ATTAAAAGGA GTGATCAAAT 5915

TTTAACATTC TCTTTAATTA ATTCATTTTT AATTTTACTT TTTTTCATTT ATTGTGCACT 5975

TACTATGTGG TACTGTGCTA TAGAGGCTTT AACATTTATA AAAACACTGT GAAAGTTGCT 6035

TCAGATGAAT ATAGGTAGTA GAACGGCAGA ACTAGTATTC AAAGCCAGGT CTGATGAATC 6095

CAAAAACAAA CACCCATTAC TCCCATTTTC TGGGACATAC TTACTCTACC CAGATGCTCT 6155

GGGCTTTGTA ATGCCTATGT AAATAACATA GTTTTATGTT TGGTTATTTT CCTATGTAAT 6215

GTCTACTTAT ATATCTGTAT CTATCTCTTG CTTTGTTTCC AAAGGTAAAC TATGTGTCTA 6275

AATGTGGGCA AAAAATAACA CACTATTCCA AATTACTGTT CAAATTCCTT TAAGTCAGTG 6335

ATAATTATTT GTTTTGACAT TAATCATGAA GTTCCCTGTG CGTACTAGGT AAACCTTTAA 6395

TAGAATGTTA ATGTTTGTAT TCATTATAAG AATTTTTGGC TGTTACTTAT TTACAACAAT 6455

ATTTCACTCT AATTAGACAT TTACTAAACT TTCTCTTGAA AACAATGCCC AAAAAAGAAC 6515

ATTAGAAGAC ACGTAAGCTC AGTTGGTCTC TGCCACTAAG ACCAGCCAAC AGAAGCTTGA 6575

TTTTATTCAA ACTTTGCATT TTAGCATATT TTATCTTGGA AAATTCAATT GTGTTGGTTT 6635

TTTGTTTTTG TTTGTATTGA ATAGACTCTC AGAAATCCAA TTGTTGAGTA AATCTTCTGG 6695

GTTTTCTAAC CTTTCTTTAG AT GTT ACC CTG TGT GAG GAG GCA TTC TTC AGG 6747

Asp Val Thr Leu Cys Glu Glu Ala Phe Phe Arg

180 185

TTT GCT GTT CCT ACA AAG TTT ACG CCT AAC TGG CTT AGT GTC TTG GTA 6795

Phe Ala Val Pro Thr Lys Phe Thr Pro Asn Trp Leu Ser Val Leu Val

190 195 200

GAC AAT TTG CCT GGC ACC AAA GTA AAC GCA GAG AGT GTA GAG AGG ATA 6843

Asp Asn Leu Pro Gly Thr Lys Val Asn Ala Glu Ser Val Glu Arg Ile

                205                 210                 215

AAA CGG CAA CAC AGC TCA CAA GAA CAG ACT TTC CAG CTG CTG AAG TTA 6891
Lys Arg Gln His Ser Ser Gln Glu Gln Thr Phe Gln Leu Leu Lys Leu
220 225 230 235

TGG AAA CAT CAA AAC AAA GAC CAA GAT ATA GTC AAG AAG ATC ATC CAA G 6940
Trp Lys His Gln Asn Lys Asp Gln Asp Ile Val Lys Lys Ile Ile Gln
240 245 250

GTATGATAAT CTAAAATAAA AAGATCAATC AGAAATCAAA GACACCTATT TATCATAAAC 7000
CAGGAACAAG ACTGCATGTA TGTTTAGTTG TGTGGATCTT GTTTCCCTGT TGGAATCATT 7060
GTTGGACTGA AAAAGTTTCC ACCTGATAAT GTAGATGTGA TTCCACAAAC AGTTATACAA 7120
GGTTTTGTTC TCACCCCTGC TCCCCAGTTT CCTTGTAAAG TATGTTGAAC ACTCTAAGAG 7180
AAGAGAAATG CATTTGAAGG CAGGGCTGTA TCTCAGGGAG TCGCTTCCAG ATCCCTTAAC 7240
GCTTCTGTAA GCAGCCCCTC TAGACCACCA AGGAGAAGCT CTATAACCAC TTTGTATCTT 7300
ACATTGCACC TCTACCAAGA AGCTCTGTTG TATTTACTTG GTAATTCTCT CCAGGTAGGC 7360
TTTTCGTAGC TTACAAATAT GTTCTTATTA ATCCTCATGA TATGGCCTGC ATTAAAATTA 7420
TTTTAATGGC ATATGTTATG AGAATTAATG AGATAAAATC TGAAAAGTGT TTGAGCCTCT 7480
TGTAGGAAAA AGCTAGTTAC AGCAAAATGT TCTCACATCT TATAAGTTTA TATAAAGATT 7540
CTCCTTTAGA AATGGTGTGA GAGAGAAACA GAGAGAGATA GGGAGAGAAG TGTGAAAGAA 7600
TCTGAAGAAA AGGAGTTTCA TCCAGTGTGG ACTGTAAGCT TTACGACACA TGATGGAAAG 7660
AGTTCTGACT TCAGTAAGCA TTGGGAGGAC ATGCTAGAAG AAAAAGGAAG AAGAGTTTCC 7720
ATAATGCAGA CAGGGTCAGT GAGAAATTCA TTCAGGTCCT CACCAGTAGT TAAATGACTG 7780
TATAGTCTTG CACTACCCTA AAAAACTTCA AGTATCTGAA ACCGGGGCAA CAGATTTTAG 7840
GAGACCAACG TCTTTGAGAG CTGATTGCTT TTGCTTATGC AAAGAGTAAA CTTTTATGTT 7900
TTGAGCAAAC CAAAAGTATT CTTTGAACGT ATAATTAGCC CTGAAGCCGA AAGAAAAGAG 7960
AAAATCAGAG ACCGTTAGAA TTGGAAGCAA CCAAATTCCC TATTTTATAA ATGAGGACAT 8020

TTTAACCCAG AAAGATGAAC CGATTTGGCT TAGGGCTCAC AGATACTAAG TGACTCATGT 8080
CATTAATAGA AATGTTAGTT CCTCCCTCTT AGGTTTGTAC CCTAGCTTAT TACTGAAATA 8140
TTCTCTAGGC TGTGTGTCTC CTTTAGTTCC TCGACCTCAT GTCTTTGAGT TTTCAGATAT 8200
CCTCCTCATG GAGGTAGTCC TCTGGTGCTA TGTGTATTCT TTAAAGGCTA GTTACGGCAA 8260
TTAACTTATC AACTAGCGCC TACTAATGAA ACTTTGTATT ACAAAGTAGC TAACTTGAAT 8320
ACTTTCCTTT TTTTCTGAAA TGTTATGGTG GTAATTTCTC AAACTTTTTC TTAGAAAACT 8380
GAGAGTGATG TGTCTTATTT TCTACTGTTA ATTTTCAAAA TTAGGAGCTT CTTCCAAAGT 8440
TTTGTTGGAT GCCAAAAATA TATAGCATAT TATCTTATTA TAACAAAAAA TATTTATCTC 8500
AGTTCTTAGA AATAAATGGT GTCACTTAAC TCCCTCTCAA AAGAAAAGGT TATCATTGAA 8560
ATATAATTAT GAAATTCTGC AAGAACCTTT TGCCTCACGC TTGTTTTATG ATGGCATTGG 8620
ATGAATATAA ATGATGTGAA CACTTATCTG GGCTTTTGCT TTATGCAG AT ATT GAC 8676
Asp Ile Asp

CTC TGT GAA AAC AGC GTG CAG CGG CAC ATT GGA CAT GCT AAC CTC ACC 8724
Leu Cys Glu Asn Ser Val Gln Arg His Ile Gly His Ala Asn Leu Thr
255 260 265 270

TTC GAG CAG CTT CGT AGC TTG ATG GAA AGC TTA CCG GGA AAG AAA GTG 8772
Phe Glu Gln Leu Arg Ser Leu Met Glu Ser Leu Pro Gly Lys Lys Val
275 280 285

GGA GCA GAA GAC ATT GAA AAA ACA ATA AAG GCA TGC AAA CCC AGT GAC 8820
Gly Ala Glu Asp Ile Glu Lys Thr Ile Lys Ala Cys Lys Pro Ser Asp
290 295 300

CAG ATC CTG AAG CTG CTC AGT TTG TGG CGA ATA AAA AAT GGC GAC CAA 8868

Gln Ile Leu Lys Leu Leu Ser Leu Trp Arg Ile Lys Asn Gly Asp Gln
305 310 315

GAC ACC TTG AAG GGC CTA ATG CAC GCA CTA AAG CAC TCA AAG ACG TAC 8916
Asp Thr Leu Lys Gly Leu Met His Ala Leu Lys His Ser Lys Thr Tyr
                320                 325                 330

CAC TTT CCC AAA ACT GTC ACT CAG AGT CTA AAG AAG ACC ATC AGG TTC 8964
His Phe Pro Lys Thr Val Thr Gln Ser Leu Lys Lys Thr Ile Arg Phe
335 340 345 350

CTT CAC AGC TTC ACA ATG TAC AAA TTG TAT CAG AAG TTA TTT TTA GAA 9012
Leu His Ser Phe Thr Met Tyr Lys Leu Tyr Gln Lys Leu Phe Leu Glu
355 360 365

ATG ATA GGT AAC CAG GTC CAA TCA GTA AAA ATA AGC TGC TTA 9054
Met Ile Gly Asn Gln Val Gln Ser Val Lys Ile Ser Cys Leu
370 375 380

TAACTGGAAA TGGCCATTGA GCTGTTTCCT CACAATTGGC GAGATCCCAT GGATGAGTAA 9114
ACTGTTTCTC AGGCACTTGA GGCTTTCAGT GATATCTTTC TCATTACCAG TGACTAATTT 9174
TGCCACAGGG TACTAAAAGA AACTATGATG TGGAGAAAGG ACTAACATCT CCTCCAATAA 9234
ACCCCAAATG GTTAATCCAA CTGTCAGATC TGGATCGTTA TCTACTGACT ATATTTTCCC 9294
TTATTACTGC TTGCAGTAAT TCAACTGGAA ATTAAAAAAA AAAAACTAGA CTCCACTGGG 9354
CCTTACTAAA TATGGGAATG TCTAACTTAA ATAGCTTTGG GATTCCAGCT ATGCTAGAGG 9414
CTTTTATTAG AAAGCCATAT TTTTTTCTGT AAAAGTTACT AATATATCTC TAACACTATT 9474

ACAGTATTGC TATTTATATT CATTCAGATA TAAGATTTGG ACATATTATC ATCCTATAAA 9534
GAAACGGTAT GACTTAATTT TAGAAAGAAA ATTATATTCT GTTTATTATG ACAAATGAAA 9594
GAGAAAATAT ATATTTTTAA TGGAAACTTT GTAGCATTTT TCTAATAGGT ACTGCCATAT 9654
TTTTCTGTGT GGAGTATTTT TATAATTTTA TCTGTATAAG CTGTAATATC ATTTTATAGA 9714
AAATGCATTA TTTAGTCAAT TGTTTAATGT TGGAAAACAT ATGAAATATA AATTATCTGA 9774
ATATTAGATG CTCTGAGAAA TTGAATGTAC CTTATTTAAA AGATTTTATG GTTTTATAAC 9834
TATATAAATG ACATTATTAA AGTTTTCAAA TTATTTTTTA TTGCTTTCTC TGTTGCTTTT 9894
ATTT 9898

Sequence number: 3
Length of sequence: 401
Sequence Type: amino acid
Strandedness: single stranded
Topology: linear
Molecular type: protein

Sequence:
Met Asn Asn Leu Leu Cys Cys Ala Leu Val Phe Leu Asp Ile Ser
-20 -15 -10
Ile Lys Trp Thr Thr Gln Glu Thr Phe Pro Pro Lys Tyr Leu His
-5 1 5
Tyr Asp Glu Glu Thr Ser His Gln Leu Leu Cys Asp Lys Cys Pro
10 15 20
Pro Gly Thr Tyr Leu Lys Gln His Cys Thr Ala Lys Trp Lys Thr
25 30 35
Val Cys Ala Pro Cys Pro Asp His Tyr Tyr Thr Asp Ser Trp His
40 45 50

Ile Gln Asp Ile Asp Leu Cys Glu Asn Ser Val Gln Arg His Ile
250 255 260
Gly His Ala Asn Leu Thr Phe Glu Gln Leu Arg Ser Leu Met Glu
265 270 275
Ser Leu Pro Gly Lys Lys Val Gly Ala Glu Asp Ile Glu Lys Thr
280 285 290
Ile Lys Ala Cys Lys Pro Ser Asp Gln Ile Leu Lys Leu Leu Ser
295 300 305
Leu Trp Arg Ile Lys Asn Gly Asp Gln Asp Thr Leu Lys Gly Leu
310 315 320
Met His Ala Leu Lys His Ser Lys Thr Tyr His Phe Pro Lys Thr
325 330 335
Val Thr Gln Ser Leu Lys Lys Thr Ile Arg Phe Leu His Ser Phe
340 345 350
Thr Met Tyr Lys Leu Tyr Gln Lys Leu Phe Leu Glu Met Ile Gly
355 360 365
Asn Gln Val Gln Ser Val Lys Ile Ser Cys Leu
370 375 380
Sequence number: 4
Length of sequence: 1206
Sequence Type: nucleic acid
Strandedness: single stranded
Topology: linear
Molecular type: cDNA

Sequence:
ATGAACAACT TGCTGTGCTG CGCGCTCGTG TTTCTGGACA TCTCCATTAA GTGGACCACC 60
CAGGAAACGT TTCCTCCAAA GTACCTTCAT TATGACGAAG AAACCTCTCA TCAGCTGTTG 120
TGTGACAAAT GTCCTCCTGG TACCTACCTA AAACAACACT GTACAGCAAA GTGGAAGACC 180
GTGTGCGCCC CTTGCCCTGA CCACTACTAC ACAGACAGCT GGCACACCAG TGACGAGTGT 240
CTATACTGCA GCCCCGTGTG CAAGGAGCTG CAGTACGTCA AGCAGGAGTG CAATCGCACC 300
CACAACCGCG TGTGCGAATG CAAGGAAGGG CGCTACCTTG AGATAGAGTT CTGCTTGAAA 360
CATAGGAGCT GCCCTCCTGG ATTTGGAGTG GTGCAAGCTG GAACCCCAGA GCGAAATACA 420
GTTTGCAAAA GATGTCCAGA TGGGTTCTTC TCAAATGAGA CGTCATCTAA AGCACCCTGT 480
AGAAAACACA CAAATTGCAG TGTCTTTGGT CTCCTGCTAA CTCAGAAAGG AAATGCAACA 540
CACGACAACA TATGTTCCGG AAACAGTGAA TCAACTCAAA AATGTGGAAT AGATGTTACC 600
CTGTGTGAGG AGGCATTCTT CAGGTTTGCT GTTCCTACAA AGTTTACGCC TAACTGGCTT 660
AGTGTCTTGG TAGACAATTT GCCTGGCACC AAAGTAAACG CAGAGAGTGT AGAGAGGATA 720
AAACGGCAAC ACAGCTCACA AGAACAGACT TTCCAGCTGC TGAAGTTATG GAAACATCAA 780
AACAAAGACC AAGATATAGT CAAGAAGATC ATCCAAGATA TTGACCTCTG TGAAAACAGC 840
GTGCAGCGGC ACATTGGACA TGCTAACCTC ACCTTCGAGC AGCTTCGTAG CTTGATGGAA 900
AGCTTACCGG GAAAGAAAGT GGGAGCAGAA GACATTGAAA AAACAATAAA GGCATGCAAA 960
CCCAGTGACC AGATCCTGAA GCTGCTCAGT TTGTGGCGAA TAAAAAATGG CGACCAAGAC 1020
ACCTTGAAGG GCCTAATGCA CGCACTAAAG CACTCAAAGA CGTACCACTT TCCCAAAACT 1080
GTCACTCAGA GTCTAAAGAA GACCATCAGG TTCCTTCACA GCTTCACAAT GTACAAATTG 1140
TATCAGAAGT TATTTTTAGA AATGATAGGT AACCAGGTCC AATCAGTAAA AATAAGCTGC 1200
TTATAA 1206

## Claims

1. A DNA comprising the nucleotide sequences of the Sequences No. 1 and No. 2 in the Sequence Table.

2. The DNA according to claim 1, wherein the Sequence ID No. 1 includes the first exon of the OCIF gene and the Sequence ID No. 2 includes the second, third, fourth, and fifth exons.

3. A protein exhibiting the activity of inhibiting differentiation and/or maturation of osteoclasts and having the following physicochemical characteristics,
(a) molecular weight (SDS-PAGE):
(i) Under reducing conditions: about 60 kD,
(ii) Under non-reducing conditions: about 60 kD and about 120 kD;
(b) amino acid sequence:
includes an amino acid sequence of the Sequence ID No. 3 in the Sequence Table,
(c) affinity:
exhibits affinity to a cation exchanger and heparin, and
(d) heat stability:
(i) the osteoclastogenesis-inhibitory activity is reduced when treated with heat at 70°C for 10 minutes or at 56°C for 30 minutes,
(ii) the osteoclastogenesis-inhibitory activity is lost when treated with heat at 90°C for 10 minutes.

4. A process for producing a protein exhibiting an activity of inhibiting differentiation and/or maturation of osteoclasts and having the following physicochemical characteristics,
(a) molecular weight (SDS-PAGE):
(i) Under reducing conditions: about 60 kD,
(ii) Under non-reducing conditions: about 60 kD and about 120 kD;
(b) amino acid sequence:
includes an amino acid sequence of the Sequence ID No. 3 of the Sequence Table,
(c) affinity:
exhibits affinity to a cation exchanger and heparin, and
(d) heat stability:
(i) the osteoclastogenesis-inhibitory activity is reduced when treated with heat at 70°C for 10 minutes or at 56°C for 30 minutes,
(ii) the osteoclastogenesis-inhibitory activity is lost when treated with heat at 90°C for 10 minutes,
the process comprising inserting a DNA including the nucleotide sequences of the sequences No. 1 and No. 2 in the Sequence Table into an expression vector, producing a vector capable of expressing a protein having the above-mentioned physicochemical characteristics and exhibiting the activity of inhibiting differentiation and/or maturation of osteoclasts, and producing this protein by a genetic engineering technique.
